(19)

**Europäisches Patentamt**
**European Patent Office**
**Office européen des brevets**

(11) **EP 4 733 373 A1**

(12) **EUROPEAN PATENT APPLICATION**
published in accordance with Art. 153(4) EPC

(43) Date of publication:
**29.04.2026 Bulletin 2026/18**

(21) Application number: **24825982.2**

(22) Date of filing: **20.06.2024**

(51) International Patent Classification (IPC):
*C11B 7/00* (2006.01)        *A23D 9/02* (2006.01)
*A23L 33/12* (2016.01)        *A61K 8/37* (2006.01)
*A61K 31/232* (2006.01)       *B01D 15/32* (2006.01)
*C11B 3/10* (2006.01)         *G01N 30/46* (2006.01)
*G01N 30/84* (2006.01)        *G01N 30/86* (2006.01)
*G01N 30/88* (2006.01)

(52) Cooperative Patent Classification (CPC):
**A23D 9/02; A23L 33/12; A61K 8/37; A61K 31/232;**
**B01D 15/32; C11B 3/10; C11B 7/00; G01N 30/46;**
**G01N 30/84; G01N 30/86; G01N 30/88**

(86) International application number:
**PCT/JP2024/022429**

(87) International publication number:
**WO 2024/262581 (26.12.2024 Gazette 2024/52)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **22.06.2023 JP 2023102401**

(71) Applicant: **Nissui Corporation**
**Tokyo 105-8676 (JP)**

(72) Inventors:
• **YAMAGUCHI, Hideaki**
**Hachioji-shi, Tokyo 192-0991 (JP)**
• **FURIHATA, Kiyomi**
**Hachioji-shi, Tokyo 192-0991 (JP)**
• **SATO, Seizo**
**Hachioji-shi, Tokyo 192-0991 (JP)**

(74) Representative: **Mewburn Ellis LLP**
**Aurora Building**
**Counterslip**
**Bristol BS1 6BX (GB)**

(54) **METHOD FOR PRODUCING HIGHLY UNSATURATED FATTY ACID ESTER COMPOSITION**

(57)    To provide a method for efficiently producing a target polyunsaturated fatty acid ester composition having high purity. Provided is a method for producing a polyunsaturated fatty acid ester composition from a fatty oil composition, the method including: subjecting the fatty oil composition to a first column chromatography to remove all or part of at least one component, wherein a retention time at a peak top of the at least one component corresponds to a relative value of 1.8 to 3.5, wherein the relative value is based on a retention time at a peak top of a target substance in the first column chromatography which is set to 1.0,; and subjecting an eluate containing the target substance obtained by the first column chromatography to a second column chromatography to remove all or part of at least one component , wherein a retention time at a peak top of the at least one component corresponds to a relative value of 0.90 to 1.1, wherein the relative value is based on a retention time at a peak top of the target substance in the second column chromatography which is set to 1.0.

EP 4 733 373 A1

## Description

TECHNICAL FIELD

The present invention relates to a method for producing a polyunsaturated fatty acid (PUFA) ester composition by using column chromatography.

BACKGROUND ART

Polyunsaturated fatty acids are used as pharmaceutical products and/or health foods. The polyunsaturated fatty acids are produced from polyunsaturated fatty acid-rich natural raw materials such as vegetable oils and marine oils. The natural raw materials also contain other components such as fatty acids with different numbers of double bonds (e.g., saturated fatty acids, monounsaturated fatty acids, fatty acids with different carbon chain lengths). The content of polyunsaturated fatty acids in natural raw materials is not high. In order to use polyunsaturated fatty acids as pharmaceutical products and/or health foods, it is necessary to selectively separate a target polyunsaturated fatty acid (PTL 1).
Column chromatography is known as a method of separating a polyunsaturated fatty acid (PTL 2). Meanwhile, pseudo-moving bed chromatography has been used as chromatography allowing for continuous operation (PTL 3-PTL 7).

CITATION LIST

PATENT LITERATURE

PTL 1: International Publication No. WO 2014/054435
PTL 2: Japanese Translation of PCT International Application Publication No. 2014-511406
PTL 3: Japanese Translation of PCT International Application Publication No. H08-512336
PTL 4: Japanese Patent Laid-Open No. H08-218091
PTL 5: Japanese Translation of PCT International Application Publication No. 2016-508156
PTL 6: Japanese Patent Laid-Open No. 2019-207234
PTL 7: Japanese Translation of PCT International Application Publication No. 2013-516398

SUMMARY OF INVENTION

TECHNICAL PROBLEM

Pseudo-moving bed chromatography requires a complex system and equipment and is more expensive than chromatography using a single column. Thus, there is a need for a method that efficiently produces a target polyunsaturated fatty acid ester composition at high purity without the need for complex equipment such as pseudo-moving bed chromatography.

SOLUTION TO PROBLEM

The inventors of the present invention have unexpectedly found that a multi-stage separation method using a first column chromatography and a second column chromatography can efficiently produce a target polyunsaturated fatty acid in view of the above problem.
The present invention is directed to the following method for producing a polyunsaturated fatty acid ester composition from a fatty oil composition.

[1-1] A method for producing a polyunsaturated fatty acid ester composition from a fatty oil composition, the method including:

(a) subjecting the fatty oil composition to a first column chromatography using a first column to obtain an eluent containing the target substance, wherein all or part of at least one component is removed by the first column chromatography, wherein a retention time at a peak top of the at least one component corresponds to a relative value of 1.8 to 3.5, wherein the relative value is based on a retention time at a peak top of a target substance in the first column chromatography which is set to 1.0, wherein the target substance is a polyunsaturated fatty acid ester;
(b) subjecting the eluent containing the target substance obtained by the first column chromatography to a second column chromatography using a second column to obtain an eluent, wherein all or part of at least one component is removed by the second column chromatography, wherein a retention time at a peak top of the at least one component corresponds to a relative value of 0.90 to 1.1, wherein the relative value is based on a retention time at a peak top of the target substance in the second column chromatography which is set to 1.0; and

(c) concentrating the eluent obtained by the second column chromatography to obtain a composition containing the target substance such that the obtained composition contains the target substance in an amount of 95 wt% or higher.

[1-2] The method according to [1-1], wherein a loading interval is shorter than in a case of column chromatography using a single column with a length corresponding to a sum of lengths of the first and second columns, wherein the loading interval is a period from starting one run of column chromatography to reaching a state wherein starting a subsequent run of column chromatography becomes possible.

[1-3] The method according to [1-2], wherein the loading interval of column chromatography is shorter than in a case of column chromatography using only the second column, wherein the loading interval is a period from starting one run of column chromatography to reaching a state wherein starting a subsequent run of column chromatography becomes possible.

[1-4] The method according to any one of [1-1] to [1-3], wherein the at least one component having the retention time at the peak top corresponding to a relative value of 1.8 to 3.5 is at least one component other than the polyunsaturated fatty acid ester in the fatty oil composition, wherein the relative value is based on a retention time at a peak top of the target substance in the first column chromatography which is set to 1.0.

[1-5] The method according to any one of [1-1] to [1-4], wherein all or part of the at least one component that is removed by subjecting the eluent containing the target substance obtained by the first column chromatography to a second column chromatography using a second column is at least one component having the retention time at the peak top corresponding to a relative value of 0.93 to 1.09, wherein the relative value is based on a retention time at a peak top of the target substance in the second column chromatography which is set to 1.0.

[1-6] The method according to any one of [1-1] to [1-5], wherein the first column is capable of being connected with the second column to inject the eluent from the first column into the second column; and

at least one component contained in the eluent when the eluent from the first column is injected into the second column has a retention time at a peak top corresponding to a relative value in a range of 1.1 or less, wherein the relative value is based on a retention time at a peak top of the target substance in the second column chromatography which is set to 1.0.

[1-7] The method according to [1-6], wherein the at least one component contained in the eluent when the eluent from the first column is injected into the second column has a retention time at a peak top corresponding to a relative value in a range from 0.90 to 1.1, wherein the relative value is based on a retention time at a peak top of the target substance in the second column chromatography which is set to 1.0.

[1-8] The method according to [1-6], wherein the at least one component contained in the eluent has a retention time at a peak top corresponding to a relative value in a range from 0.94 to 1.1 , wherein the relative value is based on a retention time at a peak top of the target substance in the second column chromatography which is set to 1.0.

[1-9] The method according to [1-6], wherein the at least one component contained in the eluent has a retention time at a peak top corresponding to a relative value in a range from 0.96 to 1.1 , wherein the relative value is based on a retention time at a peak top of the target substance in the second column chromatography which is set to 1.0.

[1-10] The method according to [1-6], wherein the at least one component contained in the eluent has a retention time at a peak top corresponding to a relative value in a range of from 0.93 to 1.09, wherein the relative value is based on a retention time at a peak top of the target substance in the second column chromatography which is set to 1.0.

[1-11] The method according to [1-6], comprising discharging the eluent from the first column, wherein the eluent from the first column has a retention time corresponding to a relative value of 3.5 or more, wherein the relative value is based on a retention time at a peak top of the target substance in the first column which is set to 1.0, and injecting a new mobile phase into the second column.

[1-12] The method according to any one of [1-1] to [1-11], wherein the first column chromatography and the second column chromatography are each reversed-phase column chromatography.

[1-13] The method according to any one of [1-1] to [1-12], wherein the first column chromatography and the second column chromatography are each fixed-bed column chromatography.

[1-14] The method according to any one of [1-1] to [1-13], wherein the first column chromatography and the second column chromatography use an identical mobile phase.

[1-15] The method according to any one of [1-1] to [1-14], wherein the second column has a larger amount of packed stationary phase than the first column.

[1-16] The method according to any one of [1-1] to [1-15], wherein the second column has the same inner diameter as the first column and is longer than the first column.

[1-17] The method according to any one of [1-1] to [1-16], wherein the fatty oil composition is derived from any of fish oil, vegetable oil, algae, or microorganisms.

[1-18] The method according to any one of [1-1] to [1-17], wherein the target substance is an ester of any of eicosapentaenoic acid (EPA), dihomo-γ-linolenic acid (DGLA), or arachidonic acid (ARA).

[1-19] The method according to any one of [1-1] to [1-18], wherein the at least one component, all or part of which is removed by the first column chromatography, is an ester of any of C22:0, C20:0, C18:0, or C20:1.

[1-20] The method according to [1-18] or [1-19], wherein the ester is an ethyl ester.

[1-21] The method according to any one of [1-1] to [1-20], wherein the at least one component, all or part of which is removed by the first column chromatography, is present in an amount of 0.3 wt% or less based on a whole fatty acid ester contained in an eluate containing the target substance subjected to the second chromatography.

[1-22] The method according to [1-21], wherein an eluate containing the target substance subjected to the second chromatography contains the at least one component, all or part of which is removed by the first column chromatography, in an amount of 0.1 wt% or less or 0.05 wt% or less based on a whole fatty acid ester contained in the eluate.

[1-23] The method according to any one of [1-1] to [1-22], wherein the polyunsaturated fatty acid ester composition obtained in step (c) contains the target substance in an amount of 96 wt% or more.

[1-24] The method according to any one of [1-1] to [1-23], further including preparing the fatty oil composition by distillation from a raw material composition.

[1-25] The method according to any one of [1-1] to [1-24], wherein steps (a), (b) and (c) are repeated twice or more.

[1-26] The method according to any one of [1-1] to [1-25], further including subjecting the fatty oil composition to a column chromatography using the first column to determine the retention time at the peak top of the target substance prior to step (a).

[1-27] The method according to any one of [1-1] to [1-26], wherein the eluent containing the target substance obtained by the first column chromatography is an eluent from the first column until an end time of elution of the target substance.

[2-1] A method for producing an eicosapentaenoic acid ester composition from a fatty oil composition, the method including:

(a) subjecting the fatty oil composition to first column chromatography using a first column to obtain an eicosapentaenoic acid ester-containing eluent containing 0.3 wt% or less of C20:0 ester based on a whole fatty acid ester contained in the eluent;
(b) subjecting the eicosapentaenoic acid ester-containing eluent obtained by the first column chromatography to a second column chromatography using a second column to obtain an eluent; and
(c) concentrating an eluent obtained by the second column chromatography to obtain a composition containing the eicosapentaenoic acid ester such that the obtained composition contains the eicosapentaenoic acid ester in an amount of 96.5 wt% or more and at least one component other than eicosapentaenoic acid ester, and the at least one component other than eicosapentaenoic acid ester contains an ester of stearidonic acid, C19:4, or C21:5 each in an amount of 0.2 wt% or less.

[2-2] The method according to [2-1], where the obtained composition contains an ester of stearidonic acid, C19:4, or C21:5 each in an amount of 0.01 wt% to 0.2 wt % .

[2-3] The method according to [2-1] or [2-2], wherein a loading interval is shorter than in a case of column chromatography using a single column with a length corresponding to a sum of lengths of the first and second columns, wherein the loading interval is a period from starting one run of column chromatography to reaching a state wherein starting a subsequent run of column chromatography becomes possible.

[2-4] The method according to [2-3], wherein the loading interval is shorter than in a case of column chromatography using only the second column, wherein the loading interval is a period from starting one run of column chromatography to reaching a state wherein starting a subsequent run of column chromatography becomes possible.

[2-5] The method according to any one of [2-1] to [2-4], wherein at least one component having a retention time at a peak top corresponding to a relative value of 1.8 to 3.5 is at least one component other than eicosapentaenoic acid ester in the fatty oil composition, wherein the relative value is based on a retention time at a peak top of the eicosapentaenoic acid ester in the first column chromatography which is set to 1.0.

[2-6] The method according to any one of [2-1] to [2-5], wherein all or part of at least one component that is removed by subjecting an eluent containing eicosapentaenoic acid ester obtained by the first column chromatography to a second column chromatography using a second column is at least one component having a retention time at a peak top corresponding to a relative value of 0.93 to 1.09, wherein the relative value is based on a retention time at a peak top of the eicosapentaenoic acid ester in the second column chromatography which is set to 1.0.

[2-7] The method according to any one of [2-1] to [2-6], wherein the first column is capable of being connected with the second column to inject the eluent from the first column into the second column; and

the at least one component other than eicosapentaenoic acid ester contained in the eluent when the eluent from the first column is injected into the second column has a retention time at a peak top corresponding to a relative value in a range of 1.1 or less, wherein the relative value is based on a retention time at a peak top of the eicosapentaenoic acid ester in the second column chromatography which is set to 1.0.

[2-8] The method according to [2-7], wherein the at least one component other than eicosapentaenoic acid ester contained in the eluent when the eluent from the first column is injected into the second column has a retention time at a peak top corresponding to a relative value in a range from 0.90 to 1.1, wherein the relative value is based on a retention time at a peak top of the eicosapentaenoic acid ester in the second column chromatography which is set to 1.0.

[2-9] The method according to [2-7], wherein the at least one component other than eicosapentaenoic acid ester contained in the eluent when the eluent from the first column is injected into the second column has a retention time at a peak top corresponding to a relative value in a range from 0.94 to 1.1, wherein the relative value is based on a retention time at a peak top of the eicosapentaenoic acid ester in the second column chromatography which is set to 1.0.

[2-10] The method according to [2-7], wherein the at least one component other than eicosapentaenoic acid ester contained in the eluent when the eluent from the first column is injected into the second column has a retention time at a peak top corresponding to a relative value in a range from 0.96 to 1.1, wherein the relative value is based on a retention time at a peak top of the eicosapentaenoic acid ester in the second column chromatography which is set to 1.0.

[2-11] The method according to [2-7], wherein the at least one component other than eicosapentaenoic acid ester contained in the eluent has a retention time at a peak top corresponding to a relative value in a range from 0.93 to 1.09, wherein the relative value is based on a retention time at a peak top of the eicosapentaenoic acid ester in the second column chromatography which is set to 1.0.

[2-12] The method according to [2-7], comprising discharging the eluent from the first column that has a retention time corresponding to a relative value of 3.5 or more, wherein the relative value is based on a retention time at a peak top of the target substance in the first column which is set to 1.0, and injecting a new mobile phase into the second column.

[2-13] The method according to any one of [2-1] to [2-12], wherein the first column chromatography and the second column chromatography are each reversed-phase column chromatography.

[2-14] The method according to any one of [2-1] to [2-13], wherein the first column chromatography and the second column chromatography are each fixed-bed column chromatography.

[2-15] The method according to any one of [2-1] to [2-14], wherein the first column chromatography and the second column chromatography use an identical mobile phase.

[2-16] The method according to any one of [2-1] to [2-15], wherein the second column has a larger amount of packed stationary phase than the first column.

[2-17] The method according to any one of [2-1] to [2-16], wherein the second column has the same inner diameter as the first column and is longer than the first column.

[2-18] The method according to any one of [2-1] to [2-17], wherein the at least one component, all or part of which is removed by the first column chromatography, is an ester of any of C20:0, C18:0, or C20:1.

[2-19] The method according to any one of [2-1] to [2-18], wherein the fatty oil composition is derived from any of fish oil, vegetable oil, algae, or microorganisms.

[2-20] The method according to any one of [2-1] to [2-19], wherein the ester is an ethyl ester.

[2-21] The method according to any one of [2-1] to [2-20], wherein the at least one component, all or part of which is removed by the first column chromatography, is present in an amount of 0.3 wt% or less based on a whole fatty acid ester contained in an eluate containing the eicosapentaenoic acid ester subjected to the second column chromatography.

[2-22] The method according to [2-21], wherein at least one component, all or part of which is removed by the first column chromatography, is present in an amount of 0.1 wt% or less or 0.05 wt% or less based on a whole fatty acid ester contained in the eluate containing the eicosapentaenoic acid ester subjected to the second column chromatography.

[2-23] The method according to any one of [2-1] to [2-22], wherein the obtained composition contains the eicosapentaenoic acid ester in an amount of 96 wt% or more.

[2-24] The method according to any one of [2-1] to [2-22], further including preparing the fatty oil composition by distillation from a raw material composition.

[2-25] The method according to any one of [2-1] to [2-24], wherein steps (a), (b) and (c) are repeated twice or more.

[2-26] The method according to any one of [2-1] to [2-25], wherein all or part of at least one component is removed in step (b), wherein a retention time at a peak top of the at least one component corresponds to a relative value of 0.90 to 1.1, wherein the relative value is based on a retention time at a peak top of the eicosapentaenoic acid ester in the second column chromatography which is set to 1.0.

[2-27] The method according to any one of [2-1] to [2-26], further including subjecting the fatty oil composition to a column chromatography using the first column to determine the retention time at the peak top of the eicosapentaenoic acid ester prior to step (a).

[2-28] The method according to any one of [2-1] to [2-27], wherein the eluent containing the eicosapentaenoic acid ester obtained by the first column chromatography is an eluent of the first column until an end time of elution of the eicosapentaenoic acid ester.

[3-1] A method for producing a dihomo-γ-linolenic acid ester composition from a fatty oil composition, the method including:

(a) subjecting the fatty oil composition to first column chromatography using a first column to obtain a dihomo-γ-linolenic acid ester-containing eluent containing 0.5 wt% or less of C22:0 ester based on a whole fatty acid ester contained in the eluent;

(b) subjecting the dihomo-γ-linolenic acid ester-containing eluent obtained by the first column chromatography to second column chromatography using a second column to obtain an eluent; and

(c) concentrating the eluent obtained by the second column chromatography to obtain a composition containing the dihomo-γ-linolenic acid ester such that the obtained composition contains the dihomo-γ-linolenic acid ester in an amount of 95.0 wt% or more and at least one component other than dihomo-γ-linolenic acid ester, and the at least one component other than dihomo-γ-linolenic acid ester contains the C20:4n-6 ester in an amount of 0.60 wt% or less.

[3-2] The method according to [3-1], where the obtained composition contains the C20:4n-6 ester in an amount of 0.01 wt% to 0.2 wt %.

[3-3] The method according to [3-1] or [3-2], wherein a loading interval is shorter than in a case of column chromatography using a single column with a length corresponding to a sum of lengths of the first and second columns, wherein the loading interval is a period from starting one run of column chromatography to reaching a state wherein starting a subsequent run of column chromatography becomes possible.

[3-4] The method according to [3-3], wherein the loading interval is shorter than in a case of column chromatography using only the second column, wherein the loading interval is a period from starting one run of column chromatography to reaching a state wherein starting a subsequent run of column chromatography becomes possible.

[3-5] The method according to any one of [3-1] to [3-4], wherein at least one component having a retention time at a peak top corresponding to a relative value of 1.8 to 3.5 is at least one component other than the dihomo-γ-linolenic acid ester in the fatty oil composition, wherein the relative value is based on a retention time at a peak top of the dihomo-γ-linolenic acid ester in the first column chromatography which is set to 1.0.

[3-6] The method according to any one of [3-1] to [3-5], wherein all or part of at least one component that is removed by subjecting an eluent containing the target substance obtained by the first column chromatography to second column chromatography using a second column is at least one component having a retention time at a peak top corresponding to a relative value of 0.93 to 1.09, wherein the relative value is based on a retention time at a peak top of the dihomo-γ-linolenic acid ester in the second column chromatography which is set to 1.0.

[3-7] The method according to any one of [3-1] to [3-6], wherein the first column is capable of being connected with the second column to inject the eluent from the first column into the second column; and

the at least one component other than dihomo-γ-linolenic acid ester contained in the eluent when the eluent from the first column is injected into the second column has a retention time at a peak top corresponding to a relative value in a range of 1.1 or less, wherein the relative value is based on a retention time at a peak top of the dihomo-γ-linolenic acid ester in the second column chromatography which is set to 1.0.

[3-8] The method according to [3-7], wherein the at least one component other than dihomo-γ-linolenic acid ester contained in the eluent when the eluent from the first column is injected into the second column has a retention time at a peak top corresponding to a relative value in a range from 0.90 to 1.1, wherein the relative value is based on a retention time at a peak top of the dihomo-γ-linolenic acid ester in the second column chromatography which is set to 1.0.

[3-9] The method according to [3-7], wherein when the at least one component other than dihomo-γ-linolenic acid ester contained in the eluent when the from the first column is injected into the second column has a retention time at a peak top corresponding to a relative value in a range from 0.94 to 1.1 or less, wherein the relative value is based on a retention time at a peak top of the dihomo-γ-linolenic acid ester in the second column chromatography which is set to 1.0.

[3-10] The method according to [3-7], wherein the at least one component other than dihomo-γ-linolenic acid ester contained in the eluent when the eluent from the first column is injected into the second column has a retention time at a peak top corresponding to a relative value in a range from 0.96 to 1.1 , wherein the relative value is based on a retention time at a peak top of the dihomo-γ-linolenic acid ester in the second column chromatography which is set to 1.0 .

[3-11] The method according to [3-7], wherein the at least one component other than dihomo-γ-linolenic acid ester contained in the eluent when the eluent from the first column is injected into the second column has a retention time at a peak top corresponding to a relative value in a range from 0.93 to 1.09, wherein the relative value is based on a retention time at a peak top of the dihomo-γ-linolenic acid ester in the second column chromatography which is set to 1.0.

[3-12] The method according to [3-7], comprising discharging the eluent from the first column that has a retention time corresponding to a relative value is 3.5 or more, wherein the relative value is based on a retention time at a peak top of the dihomo-γ-linolenic acid ester in the first column which is set to 1.0, and injecting a new mobile phase into the second column.

[3-13] The method according to any one of [3-1] to [3-12], wherein the first column chromatography and the second column chromatography are each reversed-phase column chromatography.

[3-14] The method according to any one of [3-1] to [3-13], wherein the first column chromatography and the second column chromatography are each fixed-bed column chromatography.

[3-15] The method according to any one of [3-1] to [3-14], wherein the first column chromatography and the second column chromatography use an identical mobile phase.

[3-16] The method according to any one of [3-1] to [3-15], wherein the second column has a larger amount of packed stationary phase than the first column.

[3-17] The method according to any one of [3-1] to [3-16], wherein the second column has the same inner diameter as the first column and is longer than the first column.

[3-18] The method according to any one of [3-1] to [3-17], wherein the at least one component, all or part of which is removed by the first column chromatography, is an ester of any of C22:0, C20:0, C18:0, or C20:1.

[3-19] The method according to any one of [3-1] to [3-18], wherein the fatty oil composition is derived from any of fish oil, vegetable oil, algae, or microorganisms.

[3-20] The method according to any one of [3-1] to [3-19], wherein the ester is an ethyl ester.

[3-21] The method according to any one of [3-1] to [3-20], wherein at least one component, all or part of which is removed by the first column chromatography, is present in an amount of 0.3 wt% or less based on a whole fatty acid ester contained in an eluate containing the dihomo-γ-linolenic acid ester subjected to the second column chromatography.

[3-22] The method according to [3-21], wherein the at least one component, all or part of which is removed by the first column chromatography, is present in an amount of 0.1 wt% or less or 0.05 wt% or less based on a whole fatty acid ester contained in the eluate containing the dihomo-γ-linolenic acid ester subjected to the second column chromatography.

[3-23] The method according to any one of [3-1] to [3-22], wherein the obtained composition contains the dihomo-γ-linolenic acid ester in an amount of 96 wt% or more.

[3-24] The method according to any one of [3-1] to [3-23], wherein all or part of at least one component is removed in step (b), wherein a retention time at a peak top of the at least one component corresponds to a relative value of 0.90 to 1.1, wherein the relative value is based on a retention time at a peak top of the dihomo-γ-linolenic acid ester in the second column chromatography which is set to 1.0.

[3-25] The method according to any one of [3-1] to [3-24], further including subjecting the fatty oil composition to a column chromatography using the first column to determine the retention time at the peak top of the dihomo-γ-linolenic acid ester prior to step (a).

[3-26] The method according to any one of [3-1] to [3-25], wherein the eluent containing the target substance obtained by the first column chromatography is an eluent of the first column until an end time of elution of the dihomo-γ-linolenic acid ester.

[4-1] A method for producing an arachidonic acid ester composition from a fatty oil composition, the method including:

(a) subjecting the fatty oil composition to first column chromatography using a first column to obtain an arachidonic acid ester-containing eluent containing 0.5 wt% or less of C22:0 ester based on a whole fatty acid ester contained in the eluent;

(b) subjecting the arachidonic acid ester-containing eluent obtained by the first column chromatography to a second column chromatography using a second column to obtain an eluent; and

(c) concentrating an eluent obtained by the second column chromatography to obtain a composition containing the arachidonic acid ester such that the obtained composition contains the arachidonic acid ester in an amount of 95.0 wt% or more and at least one component other than arachidonic acid ester, and the at least one component other than arachidonic acid ester contains C18:3n-6 ester in an amount of 0.25 wt% or less.

[4-2] The method according to [4-1], where the obtained composition contains the C18:3n-6 ester in an amount of 0.01 wt% to 0.2 wt %.

[4-3] The method according to [4-1] or [4-2], wherein a loading interval is shorter than in a case of chromatography using a single column with a length corresponding to a sum of lengths of the first and second columns, wherein the loading interval is a period from starting one run of column chromatography to reaching a state wherein starting a subsequent run of column chromatography becomes possible.

[4-4] The method according to [4-3], wherein the loading interval is shorter than in a case of column chromatography using only the second column, wherein the loading interval is a period from starting one run of column chromatography to reaching a state wherein starting a subsequent run of column chromatography becomes possible.

[4-5] The method according to any one of [4-1] to [4-4], wherein at least one component having a retention time at a peak top whose relative value is 1.8 to 3.5 is at least one component other than the arachidonic acid ester in the fatty oil composition, wherein the relative value is based on a retention time at a peak top of the arachidonic acid ester in the first column chromatography which is set to 1.0.

[4-6] The method according to any one of [4-1] to [4-5], wherein all or part of at least one component that is removed by subjecting an eluent containing arachidonic acid ester obtained by the first column chromatography to a second column chromatography using a second column is at least one component having a retention time at a peak top corresponding to a relative value of 0.93 to 1.09, wherein the relative value is based on a retention time at a peak top of the arachidonic acid ester in the second column chromatography which is set to 1.0.

[4-7] The method according to any one of [4-1] to [4-6], wherein the first column is capable of being connected with the second column to inject the eluent from the first column is injected into the second column; and

the at least one component other than arachidonic acid ester contained in the eluent when the eluent from the first column is injected into the second column has a retention time at a peak top corresponding to a relative value in a range of 1.1 or less, wherein the relative value is based on a retention time at a peak top of the arachidonic acid ester in the second column chromatography which is set to 1.0.

[4-8] The method according to [4-7], wherein the at least one component other than arachidonic acid ester contained in the eluent when the eluent from the first column is injected into the second column has a retention time at a peak top corresponding to a relative value in a range from 0.90 to 1.1, wherein the relative value is based on a retention time at a peak top of the arachidonic acid ester in the second column chromatography which is set to 1.0.

[4-9] The method according to [4-7], wherein the at least one component other than arachidonic acid ester contained in the eluent when the eluent from the first column is injected into the second column has a retention time at a peak top corresponding to a relative value in a range from 0.94 to 1.1, wherein the relative value is based on a retention time at a peak top of the arachidonic acid ester in the second column chromatography which is set to 1.0.

[4-10] The method according to [4-7], wherein the at least one component other than arachidonic acid ester contained in the eluent when the eluent from the first column is injected into the second column has a retention time at a peak top corresponding to a relative value in a range from 0.96 to 1.1, wherein the relative value is based on a retention time at a peak top of the arachidonic acid ester in the second column chromatography which is set to 1.0.

[4-11] The method according to [4-7], wherein the at least one component other than arachidonic acid ester contained in the eluent when the eluent from the first column is injected into the second column has a retention time at a peak top corresponding to a relative value in a range from 0.93 to 1.09, wherein the relative value is based on a retention time at a peak top of the arachidonic acid ester in the second column chromatography which is set to 1.0.

[4-12] The method according to [4-7], comprising discharging the eluent from the first column that has a retention time corresponding to a relative value of 3.5 or more, wherein the relative value is based on a retention time at a peak top of the arachidonic acid ester in the first column is set to 1.0, and injecting a new mobile phase into the second column.

[4-13] The method according to any one of [4-1] to [4-12], wherein the first column chromatography and the second column chromatography are each reversed-phase column chromatography.

[4-14] The method according to any one of [4-1] to [4-13], wherein the first column chromatography and the second column chromatography are each fixed-bed column chromatography.

[4-15] The method according to any one of [4-1] to [4-14], wherein the first column chromatography and the second column chromatography use an identical mobile phase.

[4-16] The method according to any one of [4-1] to [4-15], wherein the second column has a larger amount of packed stationary phase than the first column.

[4-17] The method according to any one of [4-1] to [4-16], wherein the second column has the same inner diameter as the first column and is longer than the first column.

[4-18] The method according to any one of [4-1] to [4-17], wherein at least one component, all or part of which is removed by the first column chromatography, is an ester of any of C22:0, C20:0, C18:0, or C20:1.

[4-19] The method according to any one of [4-1] to [4-18], wherein the fatty oil composition is derived from any of fish oil, vegetable oil, algae, or microorganisms.

[4-20] The method according to any one of [4-1] or [4-19], wherein the ester is an ethyl ester.

[4-21] The method according to any one of [4-1] to [4-20], wherein at least one component, all or part of which is removed by the first column chromatography, is present in an amount of 0.3 wt% or less based on a whole fatty acid ester contained in an eluate containing the arachidonic acid ester subjected to the second column chromatography.

[4-22] The method according to [4-21], wherein the at least one component, all or part of which is removed by the first column chromatography, is present in an amount of 0.1 wt% or less or 0.05 wt% or less based on a whole fatty acid ester contained in an eluate containing the arachidonic acid ester subjected to the second chromatography.

[4-23] The method according to any one of [4-1] to [4-22], wherein the produced composition contains the arachidonic acid ester in an amount of 96 wt% or more.

[4-24] The method according to any one of [4-1] to [4-23], further including preparing the fatty oil composition by distillation from a raw material composition.

[4-25] The method according to any one of [4-1] to [4-24], wherein steps (a), (b) and (c) are repeated twice or more.

[4-26] The method according to any one of [4-1] to [4-25], wherein all or part of at least one component is removed in step (b), wherein a retention time at a peak top of the at least one component corresponds to a relative value of 0.90 to 1.1, wherein the relative value is based on a retention time at a peak top of the arachidonic acid ester in the second column chromatography which is set to 1.0.

[4-27] The method according to any one of [4-1] to [4-26], further including subjecting the fatty oil composition to a column chromatography using the first column to determine the retention time at the peak top of the arachidonic acid ester prior to step (a).

[4-28] The method according to any one of [4-1] to [4-27], wherein the eluent containing the target substance obtained by the first column chromatography is an eluent of the first column until an end time of elution of the arachidonic acid ester.

[5-1] A method of reducing a time required for column chromatography during production of a polyunsaturated fatty acid ester composition from a fatty oil composition, the method including:

> (a) subjecting the fatty oil composition to a first column chromatography using a first column to obtain an eluent containing the target substance, wherein all or part of at least one component is removed by the first column chromatography, wherein a retention time at a peak top of the at least one component corresponds to a relative value of 1.8 to 3.5, wherein the relative value is based on a retention time at a peak top of a target substance in the first column chromatography which is set to 1.0 , wherein the target substance is a polyunsaturated fatty acid ester;
>
> (b) subjecting an eluent containing the target substance obtained by the first column chromatography to a second column chromatography using a second column to obtain an eluent, wherein all or part of at least one component is removed by the second chromatography, wherein a retention time at a peak top of the at least one component corresponds to a relative value is 0.90 to 1.1 , wherein the relative value is based on a retention time at a peak top of the target substance in the second column chromatography which is set to 1.0; and
>
> (c) concentrating an eluent obtained by the second column chromatography to obtain a composition containing the target substance such that the obtained composition contains the target substance in an amount of 95 wt% or higher, wherein

a loading interval is shorter than in a case of column chromatography using a single column with a length corresponding to a sum of lengths of the first and second columns, wherein the loading interval is a period from starting one run of column chromatography to reaching a state wherein starting a subsequent run of column chromatography becomes possible.

[5-2] The method according to [5-1], wherein the loading interval is shorter than in a case of column chromatography using only the second column, wherein the loading interval is a period from starting one run of column chromatography to reaching a state wherein starting a subsequent run of column chromatography becomes possible.

[5-3] The method according to [5-1] or [5-2], wherein the at least one component having a retention time at a peak top corresponding to a relative value is 1.8 to 3.5 is at least one component other than the polyunsaturated fatty acid ester in the fatty oil composition, wherein the relative value is based on a retention time at a peak top of a target substance in the first column chromatography which is set to 1.0.

[5-4] The method according to any one of [5-1] to [5-3], wherein all or part of the at least one component that is removed by subjecting an eluent containing the target substance obtained by the first column chromatography to a second column chromatography using a second column is at least one component having the retention time at the peak top corresponding to a relative value of 0.93 to 1.09, wherein the relative value is based on a retention time at a peak top of the target substance in the second column chromatography which is set to 1.0.

[5-5] The method according to any one of [5-1] to [5-4], wherein the first column is capable of being connected with the second column to inject the eluent from the first column into the second column; and

at least one component contained in the eluent when the eluent from the first column is injected into the second column has a relative value of the retention time at the peak top of a component contained in the eluent is in a range of 1.1 or less, wherein the relative value is based on a retention time at a peak top of the target substance in the second column chromatography which is set to 1.0.

[5-6] The method according to [5-5], wherein at least one component contained in the eluent when the eluent from the first column is injected into the second column has a retention time at a peak top corresponding to a relative value in a range from 0.90 to 1.1, wherein the relative value is based on a retention time at a peak top of the target substance in the second column chromatography which is set to 1.0.

[5-7] The method according to [5-5], wherein the at least one component contained in the eluent when the eluent from the first column is injected into the second column has a retention time at a peak top corresponding to a retention time in a range from 0.94 to 1.1 , wherein the relative value is based on a retention time at a peak top of the target substance in the second column chromatography which is set to 1.0.

[5-8] The method according to [5-5], wherein the at least one component contained in the eluent when the eluent from the first column is injected into the second column has a retention time at a peak top corresponding to a retention time in a range from 0.96 to 1.1 , wherein the relative value is based on a retention time at a peak top of the target substance in the second column chromatography which is set to 1.0 .

[5-9] The method according to [5-5], wherein the at least one component contained in the eluent when the eluent from the first column is injected into the second column has a retention time at a peak top corresponding to a relative value in a range from 0.93 to 1.09, wherein the relative value is based on a retention time at a peak top of the target substance in the second column chromatography which is set to 1.0.

[5-10] The method according to [5-5], comprising discharging the eluent from the first column that has a retention time corresponding to a relative value is 3.5 or more, wherein the relative value is based on a retention time at a peak top of the target substance in the first column which is set to 1.0, and injecting a new mobile phase into the second column.

[5-11] The method according to any one of [5-1] to [5-10], wherein the first column chromatography and the second column chromatography are each reversed-phase column chromatography.

[5-12] The method according to any one of [5-1] to [5-11], wherein the first column chromatography and the second column chromatography are each fixed-bed column chromatography.

[5-13] The method according to any one of [5-1] to [5-12], wherein the first column chromatography and the second column chromatography use an identical mobile phase.

[5-14] The method according to any one of [5-1] to [5-13], wherein the second column has a larger amount of packed stationary phase than the first column.

[5-15] The method according to any one of [5-1] to [5-14], wherein the second column has the same inner diameter as the first column and is longer than the first column.

[5-16] The method according to any one of [5-1] to [5-15], wherein the target substance is an ester of any of eicosapentaenoic acid (EPA), dihomo-γ-linolenic acid (DGLA), or arachidonic acid (ARA).

[5-17] The method according to any one of [5-1] to [5-16], wherein the at least one component, all or part of which is removed by the first column chromatography, is an ester of any of C22:0, C20:0, C18:0, or C20:1.

[5-18] The method according to any one of [5-1] to [5-17], wherein the fatty oil composition is derived from any of fish oil, vegetable oil, algae, or microorganisms.

[5-19] The method according to [5-17] or [5-18], wherein the ester is an ethyl ester.

[5-20] The method according to any one of [5-1] to [5-19], wherein the at least one component, all or part of which is removed by the first column chromatography, is present in an amount of 0.3 wt% or less based on a whole fatty acid ester contained in an eluate containing the target substance subjected to the second column chromatography.

[5-21] The method according to [5-20], wherein the at least one component, all or part of which is removed by the first column chromatography, is present in an amount of 0.1 wt% or less or 0.05 wt% or less based on a whole fatty acid ester contained in an eluate containing the target substance subjected to the second column chromatography.

[5-22] The method according to any one of [5-1] to [5-21], wherein the polyunsaturated fatty acid ester composition obtained in step (c) contains the target substance in an amount of 96 wt% or more.

[5-23] The method according to any one of [5-1] to [5-22], further including preparing the fatty oil composition by distillation from a raw material composition.

[5-24] The method according to any one of [5-1] to [5-23], wherein steps (a), (b) and (c) are repeated twice or more.

[5-25] The method according to any one of [5-1] to [5-24], further including subjecting the fatty oil composition to a column chromatography using the first column to determine the retention time at the peak top of the target substance prior to step (a).

[5-26] The method according to any one of [5-1] to [5-25], wherein the eluent containing the target substance obtained by the first column chromatography is an eluent of the first column until an end time of elution of the target substance.

[6-1] A method for producing a polyunsaturated fatty acid ester composition from a fatty oil composition, the method including:

(a) subjecting the fatty oil composition to a first column chromatography using a first column to obtain an eluent containing the target substance, wherein all or part of at least one component is removed by the first column chromatography, wherein a retention time at a peak top of the at least one component corresponds to a relative value of 1.8 to 3.5, wherein the relative value is based on a retention time at a peak top of a target substance in the first column chromatography which is set to 1.0, wherein the target substance is a polyunsaturated fatty acid ester;

(b) subjecting the eluent containing the target substance obtained by the first column chromatography to a second column chromatography using a second column to obtain an eluent, wherein all or part of at least one component is removed by the second chromatography, wherein a retention time at a peak top of the at least one component corresponds to a relative value of 0.90 to 1.1, wherein the relative value is based on a retention time at a peak top of the target substance in the second column chromatography which is set to 1.0.

[6-2] The method according to [6-1], wherein a loading interval is shorter than in a case of column chromatography using a single column with a length corresponding to a sum of lengths of the first and second columns, wherein the loading interval is a period from starting one run of column chromatography to reaching a state wherein starting a subsequent run of column chromatography becomes possible.

[6-3] The method according to [6-2], wherein the loading interval of column chromatography is shorter than in a case of column chromatography using only the second column, wherein the loading interval is a period from starting one run of column chromatography to reaching a state wherein starting a subsequent run of column chromatography becomes possible.

[6-4] The method according to any one of [6-1] to [6-3], wherein the at least one component having the retention time at the peak top corresponding to a relative value of 1.8 to 3.5 is at least one component other than the polyunsaturated fatty acid ester in the fatty oil composition, wherein the relative value is based on a retention time at a peak top of the target substance in the first column chromatography which is set to 1.0.

[6-5] The method according to any one of [6-1] to [6-4], wherein all or part of the at least one component that is removed by subjecting the eluent containing the target substance obtained by the first column chromatography to a second column chromatography using a second column is at least one component having the retention time at the peak top corresponding to a relative value of 0.93 to 1.09 or, wherein the relative value is based on a retention time at a peak top of the target substance in the second column chromatography which is set to 1.0.

[6-6] The method according to any one of [6-1] to [6-5], wherein the first column is capable of being connected with the second column to inject the eluent from the first column into the second column; and

at least one component contained in the eluent when the eluent from the first column is injected into the second column has a retention time at a peak top corresponding to a relative value in a range of 1.1 or less, wherein the relative value is based on a retention time at a peak top of the target substance in the second column chromatography which is set to 1.0.

[6-7] The method according to [6-6], wherein the at least one component contained in the eluent when the eluent from the first column is injected into the second column has a retention time at a peak top corresponding to a relative value in a range from 0.90 to 1.1, wherein the relative value is based on a retention time at a peak top of the target substance in the second column chromatography which is set to 1.0.

[6-8] The method according to [6-6], wherein the at least one component contained in the eluent has a retention time at a peak top corresponding to a relative value in a range from 0.94 to 1.1, wherein the relative value is based on a retention time at a peak top of the target substance in the second column chromatography which is set to 1.0.

[6-9] The method according to [6-6], wherein the at least one component contained in the eluent has a retention time at a peak top corresponding to a relative value in a range from 0.96 to 1.1, wherein the relative value is based on a retention time at a peak top of the target substance in the second column chromatography which is set to 1.0.

[6-10] The method according to [6-6], wherein the at least one component contained in the eluent has a retention time at a peak top corresponding to a relative value in a range from 0.93 to 1.09, wherein the relative value is based on a retention time at a peak top of the target substance in the second column chromatography which is set to 1.0.

[6-11] The method according to [6-6], comprising discharging the eluent from the first column, wherein the eluent containing one or more components discharged from the first column has a retention time corresponding to a relative value of 3.5 or more, wherein the relative value is based on a retention time at a peak top of the target substance in the first column which is set to 1.0, and injecting a new mobile phase into the second column.

[6-12] The method according to any one of [6-1] to [6-11], wherein the first column chromatography and the second column chromatography are each reversed-phase column chromatography.

[6-13] The method according to any one of [6-1] to [6-12], wherein the first column chromatography and the second column chromatography are each fixed-bed column chromatography.

[6-14] The method according to any one of [6-1] to [6-13], wherein the first column chromatography and the second column chromatography use an identical mobile phase.

[6-15] The method according to any one of [6-1] to [6-14], wherein the second column has a larger amount of packed stationary phase than the first column.

[6-16] The method according to any one of [6-1] to [6-15], wherein the second column has the same inner diameter as the first column and is longer than the first column.

[6-17] The method according to any one of [6-1] to [6-16], wherein the fatty oil composition is derived from any of fish oil, vegetable oil, algae, or microorganisms.

[6-18] The method according to any one of [6-1] to [6-17], wherein the target substance is an ester of any of eicosapentaenoic acid (EPA), dihomo-γ-linolenic acid (DGLA), or arachidonic acid (ARA).

[6-19] The method according to any one of [6-1] to [6-18], wherein the at least one component, all or part of which is removed by the first column chromatography, is an ester of any of C22:0, C20:0, C18:0, or C20:1.

[6-20] The method according to [6-18] or [6-19], wherein the ester is an ethyl ester.

[6-21] The method according to any one of [6-1] to [6-20], wherein the at least one component, all or part of which is removed by the first column chromatography, is present in an amount of 0.3 wt% or less based on a whole fatty acid ester contained in an eluate containing the target substance subjected to the second chromatography.

[6-22] The method according to [6-21], wherein an eluate containing the target substance subjected to the second chromatography contains the at least one component, all or part of which is removed by the first column chromatography, in an amount of 0.1 wt% or less or 0.05 wt% or less based on a whole fatty acid ester contained in the eluate.

[6-23] The method according to any one of [6-1] to [6-22], wherein the composition obtained in step (b) contains the target substance in an amount of 96 wt% or more.

[6-24] The method according to any one of [6-1] to [6-23], further including preparing the fatty oil composition by distillation from a raw material composition.

[6-25] The method according to any one of [6-1] to [6-24], wherein steps (a), (b) and (c) are repeated twice or more.

[6-26] The method according to any one of [6-1] to [6-25], further including subjecting the fatty oil composition to a column chromatography using the first column to determine the retention time at the peak top of the target substance prior to step (a).

[6-27] The method according to any one of [6-1] to [6-26], wherein the eluent containing the target substance obtained by the first column chromatography is an eluent from the first column until an end time of elution of the target substance.

ADVANTAGEOUS EFFECTS OF INVENTION

In the present invention, it has been found that a polyunsaturated fatty acid ethyl ester (e.g., EPA ethyl ester, DGLA ethyl ester, ARA ethyl ester)-rich composition can be efficiently produced in a shorter time and using less solvent than when using a single column. The present invention can remove fatty acids such as C20:0 ethyl ester.

DESCRIPTION OF EMBODIMENTS

In one aspect of the invention, a "composition" may contain one or more PUFAs or ester derivatives thereof. The PUFA- or its ester derivative-containing composition can be obtained from a raw material containing polyunsaturated fatty acids as constituent fatty acids. For example, a suitable fatty oil composition may be obtained from a raw material composition containing esterified natural oils and fats, including plant and animal oils and fats, and from a raw material composition containing esterified oils obtained from genetically modified plants, animals, or microorganisms including yeast or filamentous fungi. Examples of the filamentous fungi include Mortierella sp. microorganisms, from which a composition containing esterified oils can be obtained. Examples include compositions containing ethyl-esterified fish oils, algae and microalgae oils, or plant oils such as Borago oil, Echium oil, and Oenothera oil. In one embodiment, the raw material composition is a composition containing ethyl-esterified fish oil. In another embodiment, the raw material composition is a composition containing ethyl-esterified algae oil.

In one aspect of the present invention, the fatty oil composition is obtained by subjecting a raw material composition to distillation.

In one aspect of the present invention, a polyunsaturated fatty acid ester composition is a composition obtained by the method of the present invention, and is a composition containing a polyunsaturated fatty acid ester that is a target substance. The target substance polyunsaturated fatty acid ester may be an ester (e.g., an ethyl ester) of any of eicosapentaenoic acid (EPA), dihomo-γ-linolenic acid (DGLA), or arachidonic acid (ARA).

The term "fatty acid" herein refers to long-chain aliphatic carboxylic acids (alkanoic acids) with various chain lengths from about C12 to C22 (where the number refers to the total number of carbon atoms in the chain). The chain lengths are mainly C16 to C22. The structure of a fatty acid can be denoted by the simple notation "X:Y". Here, X is the total number of carbon atoms in a particular fatty acid and Y is the number of double bonds. For example, a saturated fatty acid containing 20 carbon atoms may be represented as "C20:0", a monounsaturated fatty acid containing 18 carbon atoms as, for instance, "C18:1", and arachidonic acid as, for instance, "C20:4,n-6". The "n-" indicates the position where the double bond starts as counted from the methyl terminus of the fatty acid. For example, the "n-6" indicates that the position where the double bond starts is the sixth position as counted from the methyl terminus of the fatty acid. This method is well-known to those skilled in the art, and fatty acids denoted according to this method can be easily identified by those skilled in the art.

Fatty acids are carboxylic acids with long aliphatic chains, which can be either saturated or unsaturated. Fatty acids are produced industrially by hydrolysis of triglycerides or phospholipids, which are usually derived from natural sources. Some are produced by synthesis. Regardless of the method of production, a purification process is required to obtain a pure product for food, cosmetic, or industrial use.

As used herein, the term "polyunsaturated fatty acid (PUFA)" means a fatty acid with more than one double bond. The polyunsaturated fatty acid herein is in the form of ester. Typical examples of the ester include an alkyl ester such as a $C_1$-$C_6$ alkyl ester or a $C_1$-$C_4$ alkyl ester. Examples of the ester include an ethyl ester.

Examples of the polyunsaturated fatty acid in an aspect of the present invention include eicosapentaenoic acid, docosapentaenoic acid, docosahexaenoic acid, dihomo-γ-linolenic acid, arachidonic acid, stearidonic acid, C18:3, C19:4, C20:4, or C21:5. Examples of the polyunsaturated fatty acid in an aspect of the present invention include eicosapentaenoic acid, dihomo-γ-linolenic acid, or arachidonic acid. In an aspect of the present invention, examples of the at least one component, all or part of which is removed by the second column chromatography, include C18:3, C19:4, C20:4, or C21:5. The at least one component, all or part of which is removed by the second column chromatography, may be an analogue of the target polyunsaturated fatty acid.

As used herein, the term "eicosapentaenoic acid (EPA)" is the generic name for cis-5,8,11,14,17-eicosapentaenoic acid. This fatty acid is C20:5n-3 fatty acid. "EPA-EE" may specifically refer to EPA ethyl ester.

As used herein, the "dihomo-γ-linolenic acid (DGLA)" is the generic name for cis-8,11,14-eicosatrienoic acid; this fatty acid is C20:3n-6 fatty acid.

As used herein, the "arachidonic acid (ARA)" is the generic name for cis-5,8,11,14-eicosatetraenoic acid; this fatty acid is C20:4n-6 fatty acid.

In an aspect of the present invention, the fatty oil composition typically contains a polyunsaturated fatty acid and at least one fatty acid. Thus, the fatty oil composition more typically contains a polyunsaturated fatty acid and at least one fatty acid and/or an ester thereof. The typical fatty acid ester is likewise defined for the above-described polyunsaturated fatty acid. Preferably, the fatty oil composition contains at least one of a C20 fatty acid or fatty acid ethyl ester. More preferably, the fatty oil composition contains a polyunsaturated fatty acid and at least one fatty acid selected from the group consisting of C18:0 (stearic acid), C20:0 (arachidic acid), C20:1 (eicosenoic acid), C22:0 (behenic acid), and esters thereof. In an aspect of the present invention, the at least one component, all or part of which is removed by the first column chromatography, is at least one fatty acid ester described above.

In an aspect of the present invention, at least one component other than the target substance (i.e., the polyunsaturated fatty acid ester) contained in the fatty oil composition is less polar than the target substance.

In an aspect of the present invention, the fatty acid ester contained in an eluate subjected to the second column chromatography contains at least one component selected from the group consisting of C18:0, C20:0, C20:1, and C22:0 esters in an amount of 0.5 wt% or less, 0.3 wt% or less, 0.1 wt% or less, 0.05 wt% or less, 0.03 wt% or less, or 0.01 wt% or less based on the whole fatty acid ester contained in an eluate subjected to the second column chromatography.

As used herein, "column chromatography" refers to a process of selective retention or retardation of one or more components in a fluid solution when the fluid flows through a column containing a stationary phase(s) composed of finely divided material and/or material having capillary channels. The retention results from the relative distribution of the components in the mixture between a stationary phase and a bulk fluid phase (e.g., a mobile phase), which moves through the stationary phase. Column chromatography is used for the analysis and separation of a mixture containing two or more substances. Examples of column chromatography include preparative chromatography, analytical chromatography, high-performance liquid chromatography, pseudo-moving-bed chromatography, real moving-bed chromatography, or supercritical fluid chromatography (SFC).

The terms "nonpolar" and "polar" for describing the mobile phase or polyunsaturated fatty acid ester may be used relative to each other. For example, "nonpolar" may correspond to a solvent with the lowest polarity in the mobile phase, while "polar" may correspond to a solvent more polar than the "nonpolar" solvent in the mobile phase.

In an aspect of the present invention, column chromatography involves passing a fatty oil composition through one or more columns. Thus, the first column chromatography involves passing a fatty oil composition through a first column and removing all or part of at least one component that elutes slower (i.e., has a retention time corresponding to a relative value of greater than 1.0, where the relative value is based on a retention time at a peak top of the target substance in the first column which is set to 1.0) than the polyunsaturated fatty acid ester that is the target substance. The first column used may be a combination of multiple columns in series and/or in parallel. The second column chromatography involves passing the target substance-containing eluent obtained from the first column chromatography through a second column to remove all or part of its analogue component, thereby producing a polyunsaturated fatty acid ester composition containing the target substance in a given amount. The second column used may be a combination of multiple columns in series and/or in parallel. Any known column(s) may be used in the claimed method.

In an aspect of the invention, the eluent containing the target substance obtained by the first column chromatography is an eluent of the first column until an end time of elution of the target substance. In an aspect of the invention, a removal of all or part of at least one component in the first column chromatography is performed by collecting an eluent eluted from the first column until the end time of elution of the target substance.

The first column chromatography is performed using a first column. In an aspect of the present invention, the first column chromatography is performed to remove all or part of components, the retention time of which is longer than that of the target substance. In an aspect of the present invention, the retention time of the component, all or part of which is removed by the first column chromatography, is 1.5 or more, 1.7 or more, or 1.8 or more, or 10 or less, 8 or less, 5 or less, 3.5 or less, or 3.0 or less, or 1.5 to 10, 1.7 to 8, 1.8 to 5, 1.8 to 3.5, 1.8 to 3.0, or 1.8 to 2.5 the retention time of the target substance in the first column chromatography when the retention time of the target substance is set to 1. In an aspect of the present invention, when the at least one component is partially removed by the first column chromatography, 99% or more, 95% or more, or 90% or more of the component is removed.

The second column chromatography is performed using a second column. In an aspect of the present invention, the second column chromatography is performed to remove all or part of components, the retention time of which is close to that of the target substance. The component, the retention time of which is close to that of the target substance, may be referred to as an analogue herein. In an aspect of the present invention, the retention time of the component to be removed by the second column chromatography is 0.8 or more or 0.9 or more, or 1.5 or less, 1.3 or less, 1.2 or less, or 1.1 or less, or 0.8 to 1. 5, 0.9 to 1.3, 0.9 to 1.2, or 0.9 to 1.1 the retention time of the target substance in the second column chromatography when the retention time of the target substance is set to 1. In an aspect of the present invention, when the component, the retention time of which is close to that of the target substance in the second column chromatography, is partially removed, 5% or more, 10% or more, 15% or more, 20% or more, 30% or more, 40% or more, or 50% or more of the component is removed.

In an aspect of the present invention, a fraction that is an eluent from the first chromatography and is not subjected to the second chromatography and a fraction that is an eluent from the second chromatography and includes a fatty acid ester which is removed from the polyunsaturated fatty acid ester composition may be collected and subjected to third column chromatography. The third column chromatography is performed using a third column.

In an aspect of the present invention, the first column refers to a column used in the first column chromatography, the second column refers to a column used in the second column chromatography, and the third column means a column used in the third column chromatography. The dimensions of the column used are not particularly limited and will depend to some extent on the volume of the fatty oil composition subjected to purification. The diameter of each column is 1 mm or more, 2 mm or more, 4 mm or more, 8 mm or more, 16 mm or more, 32 mm or more, 64 mm or more, 128 mm or more, 256 mm or more, or 500 mm or more, or 4000 mm or less, 2000 mm or less, 1000 mm or less, or 500 mm or less, or 1 to 4000 mm, 2 to 2000 mm, 4 to 1000 mm, 8 to 1000 mm, 16 to 1000 mm, 32 to 500 mm, 30 to 800 mm, or 400 to 800 mm. In an aspect of the present invention, the first and second columns may have the same or different diameters. The ratio of the second column diameter to the first column diameter is set such that when the first column diameter is set to 1, the second column diameter is 0.1 or more, 0.3 or more, or 0.5 or more, or 10 or less, 3 or less, 2 or less, or 1.5 or less, or from 0.1 to 10, from 0.3 to 3, from 0.5 to 2, or from 0.5 to 1.5. In an aspect of the present invention, the first and second columns used have the same diameter. In an aspect of the present invention, when the third column chromatography is performed, the first to third columns may have the same or different diameters. The third column may have the same diameter as or a different diameter from that of the respective first or second column. In an aspect of the present invention, the second column has the same diameter as the first column and is longer than the first column. That the first and second columns are the same or have an identical diameter herein means that the ratio of the first column diameter to the second column diameter is from 0.8 to 1.2, for example, from 0.9 to 1.1 or 1.

In an aspect of the present invention, the cross sections of the first and second columns are substantially circular and the ratio of the second column cross-sectional area to the first column cross-sectional area is set such that when the first column cross-sectional area is set to 1, the second column cross-sectional area is 0.1 or more, or 0.2 or more, or 10 or less, 4 or less, or 2 or less, or from 0.1 to 10, from 0.2 to 4, or from 0.2 to 2. In an aspect of the present invention, the first and second columns used have the same cross-sectional area. In an aspect of the present invention, when the third column chromatography is performed, the first to third columns may have the same or different cross-sectional areas. The third column may have the same cross-sectional area as or a different cross-sectional area from that of the respective first or second column.

In an aspect of the present invention, the length of each column is 5 cm or more, 10 cm or more, or 20 cm or more, or 800 cm or less, 400 cm or less, 200 cm or less, 150 cm or less, or 120 cm or less, or from 5 to 800 cm, from 10 to 400 cm, from 20 to 200 cm, from 20 to 150 cm, or from 20 to 120 cm. In an aspect of the present invention, the first column is shorter than the second column. The ratio of the second column length to the first column length is set such that when the first column length is set to 1, the second column length is 0.5 or more, 1 or more, 1.1 or more, or 1.5 or more, or 10 or less, 7 or less, or 5 or less, or from 0.5 to 10, from 1 to 7, from 1.1 to 5, or from 1.5 to 5. In an aspect of the present invention, when the third column chromatography is performed, the third column may have the same length as or a different length from that of the respective first or second column. In an aspect of the present invention, the second column has the same inner diameter as the first column and is longer than the first column. The second column has the same inner diameter as the first column and is, for example, 1.1 times or more, or 1.5 times or more, or 5 times or less, 7 times or less, or 10 times or less than the length of the first column. That the first and second columns are the same or have an identical inner diameter herein means that the ratio of the first column inner diameter to the second column inner diameter is from 0.8 to 1.2, for example, from 0.9 to 1.1 or 1.

The column is cylindrical in shape with an outer diameter, an inner diameter, and a length. In a certain aspect, the column is a preparative chromatography column. The preparative chromatography column may have an inner diameter of 5 mm or larger, 10 mm or larger, 20 mm or larger, or 50 mm or larger, or 4 m or smaller, 2 m or smaller, 1 m or smaller, or 80 cm or smaller, or from about 5 mm to about 4 m, from about 10 mm to about 2 m, from about 20 mm to about 1 m, or from about 50 mm to 80 cm and have a length of from about 100 mm to about 5 m, from about 2 cm to about 2 m, or from about 10 cm to about 1.5 m. In a certain aspect, the column is an analytical chromatography column. The analytical column may have an inner diameter of from about 1 mm to about 10 cm and a length of from about 10 mm to about 500 mm. The dimensions may be selected such that the inner diameter is about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 120%, about 140%, about 160%, about 180%, about 200%, about 220%, about 240%, about 260%, about 280%, about 300%, about 320%, about 340%, about 360%, about 380%, about 400%, about 450%, or about 500% of the length. The outer diameter may be about 0.1%, about 0.5%, about 1.0%, about 1.5%, about 2.0%, about 2.5%, about 3.0%, about 3.5%, about 4.0%, about 4.5%, about 5%, about 7.5%, about 10%, about 15%, about 20%, about 25% or about 30% larger than the inner diameter.

As used herein, the term "peak" means a peak in a chromatogram. A chromatogram is a diagram showing detected values over time. When an eluate is detected, the detected value increases and the chromatogram shows a "peak". Each peak in the chromatogram indicates the presence of a component in a sample. Each peak is labeled with a retention time, and the time in the chromatogram increases from left to right.

The "peak top" refers to the point in the chromatogram where the detected value of each peak is the largest.

As used herein, the "separation" means a process characterized by the spatial separation of components in a composition containing a polyunsaturated fatty acid ester on the basis of their distribution difference between phases (e.g., mobile and stationary phases) in relative motion. The separation results from loading of a sample onto a column and elution from the column after the loading.

The "fractionation" is a separation process in which a given quantity of a mixture is divided into a number of smaller quantities during a phase transition, where the composition changes according to a gradient. Different fractions are collected at different time points based on the specific properties of the individual components (e.g., a polyunsaturated fatty acid ester in the mixture or sample), for instance, a difference in their affinity for the stationary and/or mobile phase.

As used herein, the wording "subjected to column chromatography" means to load a sample containing a polyunsaturated fatty acid ester onto a column until the entire sample is contained within the column. The sample herein is a fatty oil composition. The sample is typically loaded onto the top of the stationary phase in a packed column. Here, the "top" is the end of the stationary phase that first receives the mobile phase while the mobile phase elutes by passing through the column. The fatty oil composition may be subjected to column chromatography by using an injector or pump or by direct application of the sample to the top of the stationary phase. The sample may be mixed with a minimal amount of mobile phase or other solvent for loading.

As used herein, the term "elute" means that components loaded on the column pass through the column and flows out as a mobile phase solution.

As used herein, the term "eluent" refers to a mobile phase eluted from the column and may include a component(s) contained in the fatty oil composition loaded onto the column. In one aspect, the term refers to a solution of the mobile phase eluted from the column containing a component(s) contained in the fatty oil composition. In an aspect of the present invention, the wording "eluent containing the target substance obtained by the first column chromatography" means the eluent that has passed through the first column and contains the target substance.

As used herein, the term "eluate" refers to a component(s) discharged from the column in column chromatography. In an aspect of the present invention, the "eluate subjected to a second column chromatography" is an eluate of the first column chromatography.

The "component" means a component contained in a fatty oil composition subjected to column chromatography, and includes a polyunsaturated fatty acid ester and a fatty acid ester. In an aspect of the present invention, the "at least one component, all or part of which is removed by first column chromatography" may be a fatty acid ester having a longer retention time than the target substance polyunsaturated fatty acid ester, and may be, for example, an ester of any of C22:0, C20:0, C18:0 or C20:1. The "at least one component in the second column chromatography" is a component contained in the polyunsaturated fatty acid ester composition obtained from the fatty oil composition that is other than the target polyunsaturated fatty acid ester, and may be an ester of any of stearidonic acid, C19:4, C21:5, C20:4, or C18:3.

In an aspect of the present invention, the "at least one component, all or part of which is removed by first column chromatography" and the "at least one component in the second column chromatography" may differ. In an aspect of the present invention, the "at least one component in the second column chromatography" may contain a same component as "at least one component, all or part of which is removed by first column chromatography."

As used herein, the "biphenyl," "C30", "C22", "C18", "C8", "C5", and "C4" each refer to a functional group present on the column packing material (stationary phase). For example, in a biphenyl column, a material flowing through the column is exposed to unsubstituted biphenyl groups, while in a C18 column, a material (e.g., mobile phase and components) flowing through the column is exposed to unsubstituted linear or branched C18 alkyl groups.

The "chromatographic conditions" means parameters with which the column chromatography is operated. Examples include the packing pressure, compositions of mobile and stationary phases, slurry concentration, pressure at which the column is operated, column temperature, mobile phase temperature, mobile phase gradient, mobile phase flow rate, column type used, detection instrumentation and parameters used, sample preparation protocol used, settling time and pressure at which settling is performed, and standing time and pressure at which standing is performed.

The "gradient" means a change in mobile phase composition over time while column chromatography is being performed. The composition of the mobile phase may change as the solvent is eluted through the column. Different mobile phases can be added at an increasing percentage over time during elution.

The "purity" is a percentage indicating the content of the main component of a composition, and may be calculated, for example, from the results of gas chromatography (GC) measurement using an internal standard. In an aspect herein, the main component is a polyunsaturated fatty acid ester such as an EPA ester, a DGLA ester, or an ARA ester.

The "target substance" means a target polyunsaturated fatty acid ester (e.g., an EPA ester, a DGLA ester, or an ARA ester) obtained from a single fraction or a combination of fractions obtained by elution and fractionation.

In an aspect of the invention, concentration includes reducing the pressure of and/or heating an eluent comprising the target polyunsaturated fatty acid ester to recover the eluent as a solvent by distillation. In an aspect of the invention, concentration is performed by using an evaporator. The evaporator includes an eluent evaporator and/or a thin film evaporator. Any type of evaporator may be used for concentration, for example, a thin film evaporator, a multi-effect evaporation device, a multi-room evaporation device, a natural circulation evaporator, a forced circulation evaporator, a falling film evaporator, a climbing film evaporator, and a combination thereof. In an aspect of the invention, types of a natural circulation evaporator includes, for example, an external heating type, or a calandria type.

A specific range is given herein by the numerical values preceded by the term "about". The term "about" is used herein to provide textual support for the exact number following the term as well as a number that is close to or approximately that number. To determine whether a number is close to or approximately that number specifically indicated, a number that is not explicitly indicated that is close to or approximately the indicated number may be a number that provides a substantially equivalent number specifically indicated in the context where the number is given. In a certain aspect, the "about" may refer to $\pm 5\%$, $\pm 2.5\%$, or $\pm 1\%$ of the number it refers to.

In an English specification corresponding to the present specification, the use of the terms "a" and "an" and "the" and similar referents in the context of describing the elements (especially in the context of the claims) are to be construed to cover both the singular and the plural, unless otherwise indicated herein or clearly contradicted by context.

Unless otherwise defined, all technical and scientific terms used herein have the same meanings as generally understood by those skilled in the art of the present invention. Any methods and materials similar or equivalent to the methods and materials described herein may also be used in the practice or testing of the present invention. Here, representative exemplary methods and materials are described.

When a range of values is provided, it is understood that each value between the upper and lower limits of that range (up to one-tenth of a unit of the lower limit, unless the context clearly indicates otherwise) and any other stated values or intermediate values in that stated range are included within the scope of the present invention. The upper and lower limits of these smaller ranges can be independently included in the smaller ranges, and if they also include the one or both of the limits that are included in the scope of the present invention and are subject to specifically excluded limits, the ranges excluding either or both of those included limits are also included in the present invention.

The present invention is not limited to the specific aspects described (as such aspects can naturally vary). Since the scope of the present invention is considered to be limited only by the appended claims, it should also be construed that the terminology used herein is only for the purpose of describing specific aspects and is not intended to be limiting.

In an aspect of the invention, the method for producing a polyunsaturated fatty acid ester composition from a fatty oil composition comprises subjecting the fatty oil composition to a column chromatography using the first column to determine the retention time at the peak top of the target substance prior to performing the first column chromatography.

In an aspect of the present invention, all or part of at least one component is removed, wherein a retention time at a peak top of the at least one component is slower than that of the target substance of the first column chromatography, wherein the target substance is polyunsaturated fatty acid.

The "retention time" refers to the amount of time a component spends on the column after it has been injected into the column. Typically, the retention time refers to when the peak top appears in the chromatogram.

As used herein, the wording "retention time at the peak top of the target substance, wherein the target substance ispolyunsaturated fatty acid" means the retention time indicated at the peak top of the target substance in the chromatogram of the first or second chromatography.

In an aspect of the invention, the measurement result of a retention time may be used as a basis to calculate the "relative retention time (RRT)" (used interchangeable with a "relative value" or a "relative value of the retention time"), which shows the relationship of a component's retention time relative to the target substance's retention time.

As used herein, the " the retention time at the peak top corresponding to a relative value" means a value obtained by dividing the retention time at the peak top indicated by a component other than the target substance in the chromatogram of the first or second chromatography by the retention time at the peak top of the target substance when the retention time at the peak top indicated by the target substance is set to 1:

$$RRT = \frac{Retention\ time\ of\ the\ component}{Retention\ time\ of\ the\ target\ substance}.$$

An eluent having "a retention time corresponding to a relative value" that is equal to or greater than the relative retention time of the target substance is an eluent collected after the " the retention time" of the target substance. In addition, an eluent having " the retention time corresponding to a relative value" that is equal to or smaller than the relative retention time of the target substance is an eluent collected before the " the retention time" of the target substance.

In an aspect of the present invention, the retention time at the peak top can be determined by measuring the eluent with a UV detector.

In an aspect of the present invention, the retention time at the peak top can be determined by measuring each fraction by fatty acid analysis using gas chromatography.

As used herein, "one run of column chromatography" means a process in which a batch of fatty oil composition is made to pass through one or more columns to obtain a polyunsaturated fatty acid ester composition containing the target substance in a given concentration. In one run of column chromatography, a batch of fatty oil composition may be passed through one or two columns. When a batch of fatty oil composition is passed through two columns in one run of column chromatography, the two columns are called the first and second columns and are connected in series. When two columns are used in one run of column chromatography, a portion of the eluent from the first column is discharged without being injected into the second column. In an aspect of the present invention, the first column is capable of being connected with the second column so that the eluent from the first column is injected into the second column. In an aspect of the present invention, the wording "eluent from the first column is injected into the second column" means that the eluent from the first column can be applied to the second column. In an aspect, the eluent can be injected directly to the second column without concentration or other treatment. In an aspect of the present invention, the term "capable of being connected" means that the state of the first and second columns can be switched between a connected state and a non-connected state.

In an aspect of the present invention, the wording "discharging an eluent from the first column" means that the eluent from the first column is discharged as waste without being injected into the second column. In an aspect of the present invention, when the relative value of the retention time is 3.5 or more, the eluent from the first column is discharged by disconnecting the first column from the second column. In an aspect of the present invention, the eluent discharged from the first column may be injected into the third column.

In an aspect of the present invention, the wording "loading interval is a period from starting one run of column chromatography to reaching a state wherein starting a subsequent run of column chromatography becomes possible" means a duration from the time when the fatty oil composition is first loaded onto the first or second column until a state is reached wherein restarting loading of the fatty oil composition (e.g., a second loading) onto the same column in the column chromatography using a single column or two or more columns becomes possible. In this case, the second loading of the fatty oil composition onto the same column is started after an interval of time such that the elution of the target substance in the second load of a fatty oil composition begins after the elution of the last component in the one run of column chromatography is completed. In an aspect of the present invention, the "loading interval is a period from starting one run of column chromatography to reaching a state wherein starting a subsequent run of column chromatography becomes possible" is calculated or measured as a duration from the time point when elution of the target substance starts in the first or second column until the completion of elution of the last component. In an aspect of the present invention, the "loading interval is a period from starting one run of column chromatography to reaching a state wherein starting a subsequent run of column chromatography becomes possible" is calculated as the sum of the duration from the time point when the fatty oil composition is subjected to the first column chromatography until the elution of the target substance from the first column ends and the duration from the time point when elution of the target substance starts in the second column chromatography until the discharge of the last component from the second column ends. In an aspect of the present invention, the first and second columns are used, and the last component is eluted from the first or second column. In an aspect of the present invention, the "loading interval is a period from starting one run of column chromatography to reaching a state wherein starting a subsequent run of column chromatography becomes possible " is a time equal to the duration from the time point when elution of the target substance starts in the first column chromatography until the discharge of the last component from the first column ends. In another aspect of the present invention, the "loading interval is a period from starting one run of column chromatography to reaching a state wherein starting a subsequent run of column chromatography becomes possible " is the sum of the duration from the time when the fatty oil composition is subjected to the first column chromatography until the elution of the target substance from the first column ends and the duration from the time point when elution of the target substance starts in the second column chromatography until the discharge of the last component from the second column ends, or a time equal to the duration from the time point when elution of the target substance starts in the second column chromatography until the discharge of the last component from the second column ends. In an aspect of the present invention, the duration from the time point when elution of the target substance starts in the first column chromatography until the discharge of the last component from the first column ends is shorter than the duration from the time point when elution of the target substance starts in column chromatography using a single column with a length that is equal to the sum of the lengths of the first and second columns until when the discharge of the last component ends. In an aspect of the present invention, the sum of the duration from the time point when the fatty oil composition is subjected to the first column chromatography until the elution of the target substance from the first column ends and the duration from the time point when elution of the target substance starts in the first column chromatography until the discharge of the last component from the first column ends, or the duration from the time point when elution of the target substance starts in the second column chromatography until the discharge of the last component from the second column ends is shorter than the duration from the time point when elution of the target substance starts in the column chromatography using a single column with a length that is equal to the sum of the lengths of the first and second columns until the discharge of the last component ends. In addition, in another aspect of the present invention, the duration from the time point when elution of the target substance starts in the first column to the completion of elution of the last component is shorter than the duration from the time point when elution of the target substance starts in the second column in the column chromatography using only the second column until the completion of elution of the last component. Further, in an aspect of the present invention, the sum of the duration from the time point when the fatty oil composition is subjected to the first column chromatography until the elution of the target substance from the first column ends and the duration from the time point when elution of the target substance starts in the second column chromatography until the discharge of the last component from the second column ends is shorter than the duration from the time point when elution of the target substance starts in the second column in the column chromatography using only the second column until the completion of elution of the last component.

As used herein, the "single column with a length that is equal to a sum of the lengths of the first and second columns" indicates, for example, a single column having the same inner diameter and stationary phase as those of the first and/or second columns. In an aspect, the single column with a length that is equal to a sum of the lengths of the first and second columns indicates a single column having the same inner diameter and stationary phase as the second column, and a length in the flow direction that is equal to a sum of the lengths of the first and second columns.

In an aspect of the present invention, reversed-phase column chromatography is suitable for the column chromatography. Any reversed-phase distribution-based adsorbent can be used without particular limitation as the stationary phase. Examples include polymer beads made of, for instance, DVB (divinylbenzene) and reticulated polystyrene, or silica gel bonded with C4 or C8 or C18 alkyl groups, especially an ODS column using octadecylsilyl (ODS). Each chromatographic column may contain the same or different adsorbents. Typically, the first and second columns may contain the same or different adsorbents. In an aspect of the present invention, the first and second columns contain the same adsorbent. In an aspect of the present invention, when the third column chromatography is performed, the third column may have the same adsorbent as or a different adsorbent from that of the respective first or second column.

The stationary phase may be selected from C30, C22, C18, C8, C4, biphenyl, fluorophenyl, hydrophilic interaction liquid chromatography (HILIC) stationary phase, acrylamide, silica, phenylhexyl stationary phase, polar embedded alkyl, fluorophenylpropyl, or any stationary phase known in the art of chromatography.

In a certain aspect, a chiral stationary phase is used. The choice of stationary phase is obvious to those skilled in the art and may depend on the component to be purified by chromatography. A non-polar component (e.g., a polyunsaturated fatty acid ester) may require the use of a reversed phase stationary phase such as C18 (ODS). It is possible to use a variety of different types of octadecyl silica (ODS) including fully end-capped, partially end-capped, or base-inactivated ODS. More polar components may require a normal stationary phase such as non-bonded silica or amino or cyano phase.

In a certain aspect, the stationary phase contains particles with a median particle diameter of 1 $\mu$m or more, 20 $\mu$m or more, 40 $\mu$m or more, 60 $\mu$m or more, 80 $\mu$m or more, 100 $\mu$m or more, 1000 $\mu$m or more, 2000 $\mu$m or more, 3000 $\mu$m or more, or 4000 $\mu$m or more, or 20 $\mu$m or less, 40 $\mu$m or less, 60 $\mu$m or less, 80 $\mu$m or less, 100 $\mu$m or less 1000 $\mu$m or less, 2000 $\mu$m or less, 3000 $\mu$m or less, 4000 $\mu$m or less, or 5000 $\mu$m or less, or from about 1 $\mu$m to about 20 $\mu$m, from about 20 $\mu$m to about 40 $\mu$m, from about 40 $\mu$m to about 60 $\mu$m, from about 60 $\mu$m to about 80 $\mu$m, from about 80 $\mu$m to about 1000 $\mu$m, from about 1000 $\mu$m to about 2000 $\mu$m, from about 2000 $\mu$m to about 3000 $\mu$m, from about 3000 $\mu$m to about 4000 $\mu$m, or from about 4000 $\mu$m to about 5000 $\mu$m. In a certain aspect, the stationary phase contains particles with a median particle diameter larger than 50 $\mu$m, larger than 45 $\mu$m, larger than 40 $\mu$m, larger than 35 $\mu$m, larger than 30 $\mu$m, larger than 25 $\mu$m, larger than 20 $\mu$m, larger than 15 $\mu$m, or larger than 10 $\mu$m, and/or less than 5000 $\mu$m, less than 4000 $\mu$m, less than 3000 $\mu$m, less than 2000 $\mu$m, less than 1000 $\mu$m, less than 500 $\mu$m, less than 100 $\mu$m, less than 80 $\mu$m, or less than 60 $\mu$m, or from 10 $\mu$m to 5000 $\mu$m, from 15 $\mu$m to 4000 $\mu$m, from 20 $\mu$m to 3000 $\mu$m, from 25 $\mu$m to 2000 $\mu$m, from 30 $\mu$m to 1000 $\mu$m, from 35 $\mu$m to 500 $\mu$m, from 40 $\mu$m to 100 $\mu$m, from 45 $\mu$m to 80 $\mu$m, or from 50 $\mu$m to 60 $\mu$m. The median particle diameter may be measured by a laser diffraction scattering method, as understood by those skilled in the art.

In an aspect of the present invention, the same or different stationary phases may be used in the first and second columns. If different stationary phases are used, the first column may use, for example, silica gel bonded with DVB (divinylbenzene) and reticulated polystyrene, C8 alkyl groups, or octadecylsilyl (ODS); and the second column may use a different stationary phase from that of the first column, which stationary phase is selected from silica gel bonded with DVB (divinylbenzene) and reticulated polystyrene, alkyl groups, or octadecylsilyl (ODS). In an aspect of the present invention, when the third column chromatography is performed, the third column may have the same stationary phase as or a different stationary phase from that of the respective first or second column.

In addition, the first and second columns may use a stationary phase containing particles with the same or different median particle diameters. In the case of using a stationary phase containing particles with different median particle diameters, it is possible to use, for the first column, a stationary phase containing particles with a median particle diameter selected from, for example, 1 $\mu$m or more, 20 $\mu$m or more, 40 $\mu$m or more, 60 $\mu$m or more, 80 $\mu$m or more, 1000 $\mu$m or more, 2000 $\mu$m or more, 3000 $\mu$m or more, or 4000 $\mu$m or more, or 20 $\mu$m or less, 40 $\mu$m or less, 60 $\mu$m or less, 80 $\mu$m or less, 1000 $\mu$m or less, 2000 $\mu$m or less, 3000 $\mu$m or less, 4000 $\mu$m or less, or 5000 $\mu$m or less, or about 1 $\mu$m to about 20 $\mu$m, about 20 $\mu$m to about 40 $\mu$m, about 40 $\mu$m to about 60 $\mu$m, about 60 $\mu$m to about 80 $\mu$m, about 80 $\mu$m to about 1000 $\mu$m, about 1000 $\mu$m to about 2000 $\mu$m, about 2000 $\mu$m to about 3000 $\mu$m, about 3000 $\mu$m to about 4000 $\mu$m, or about 4000 $\mu$m to about 5000 $\mu$m, and it is possible to use, for the second column, a stationary phase that is different from the stationary phase for the first column and contains particles with a median particle diameter selected from 1 $\mu$m or more, 20 $\mu$m or more, 40 $\mu$m or more, 60 $\mu$m or more, 80 $\mu$m or more, 1000 $\mu$m or more, 2000 $\mu$m or more, 3000 $\mu$m or more, or 4000 $\mu$m or more, or 20 $\mu$m or less, 40 $\mu$m or less, 60 $\mu$m or less, 80 $\mu$m or less, 1000 $\mu$m or less, 2000 $\mu$m or less, 3000 $\mu$m or less, 4000 $\mu$m or less, or 5000 $\mu$m or less, or about 1 $\mu$m to about 20 $\mu$m, about 20 $\mu$m to about 40 $\mu$m, about 40 $\mu$m to about 60 $\mu$m, about 60 $\mu$m to about 80 $\mu$m, about 80 $\mu$m to about 1000 $\mu$m, about 1000 $\mu$m to about 2000 $\mu$m, about 2000 $\mu$m to about 3000 $\mu$m, about 3000 $\mu$m to about 4000 $\mu$m, or about 4000 $\mu$m to about 5000 $\mu$m. In an aspect of the present invention, when the third column chromatography is performed, the third column may use a stationary phase containing particles with the same median particle diameter as or a different median particle diameter from that of the respective first or second column.

In an aspect of the present invention, the amount of stationary phase packed is from 1 to 1000 kg, from 10 to 900 kg, from 20 to 800 kg, from 30 to 700 kg, from 40 to 600 kg, or from 50 to 500 kg by weight. In an aspect of the present invention, the amount of stationary phase in the first column and the amount of stationary phase in the second column may be the same or different. In the case of having different amounts of packed stationary phase, the amount of stationary phase in the first column may be, for example, from 1 to 100 kg, from 10 to 100 kg, or from 30 to 100 kg. In addition, the amount of stationary phase in the second column, is, for example, from 1 to 1000 kg, from 10 to 800 g, or from 50 to 800 g. The ratio of the amount of stationary phase between the first and second columns when the amount in the first column is set to 1 is from 0.5 to 10, from 1 to 7, from 1.1 to 5, or from 2 to 4.5 for the second column. In an aspect of the present invention, when the third column chromatography is performed, the third column may have a packed stationary phase in the same amount as or a different amount from that of the respective first or second column.

In an aspect of the present invention, the amount of stationary phase packed in the second column is larger than the amount of stationary phase packed in the first column. The amount of stationary phase packed in the second column is 1.1 times or more, 1.5 times or more, 2 times or more, 2.5 times or more, 3 times or more, 3.5 times or more, or 4 times or less, 4.5 times or less, 5 times or less, 6 times or less, 7 times or less, or 10 times or less than the amount of stationary phase packed in the first column.

In an aspect of the present invention, the column chromatography involves elution using a mobile phase containing one or more of water, an organic solvent, or supercritical carbon dioxide. In a method according to the aspect, the mobile phase contains methanol. In a method according to the aspect, the chromatography involves a mobile phase containing an additive selected from one or more of formic acid, ammonium formate, trimethylamine, ammonia, or ammonium hydroxide.

In an aspect of the present invention, the solvent used as the mobile phase is an organic solvent, which is selected from the group consisting of alcohols, ethers, esters, ketones, nitriles, hexanes, and dichloromethane. Examples of the alcohol include methanol, ethanol, n-propanol, i-propanol, n-butanol, i-butanol, s-butanol, or t-butanol. Methanol or ethanol is preferable. Methanol is more preferable. Examples of the ether include diethyl ether, diisopropyl ether, or methyl t-butyl ether (MTBE). Examples of the ester include methyl acetate ester and ethyl acetate ester. Examples of the ketone include acetone, methyl ethyl ketone, or methyl isobutyl ketone (MIBK). Examples of the nitrile include acetonitrile.

In an aspect of the present invention, the mobile phases of the first and second column chromatography may be the same or different, but are preferably the same. In the case of using different mobile phases, the first column chromatography may use, for example, methanol, ethanol, diethyl ether, diisopropyl ether, methyl t-butyl ether, methyl acetate, ethyl acetate, acetone, methyl ethyl ketone, methyl isobutyl ketone (MIBK), acetonitrile, hexanes, or dichloromethane; and the second column may use a mobile phase that is different from the first column mobile phase and is selected from the group consisting of methanol, ethanol, diethyl ether, diisopropyl ether, methyl t-butyl ether, methyl acetate, ethyl acetate, acetone, methyl ethyl ketone, methyl isobutyl ketone (MIBK), acetonitrile, hexanes, and dichloromethane. In an aspect of the present invention, when the third column chromatography is performed, the third column may have the same mobile phase as or a different mobile phase from that of the respective first or second column.

The mobile phase may further contain additives including a buffer and a pH adjuster. The choice of additives may be determined based on the mobile phase used, the stationary phase used, and the component(s) to be purified. In some aspects, the mobile phase contains an additive(s) selected from one or more of formic acid, trifluoroacetic acid, heptafluorobutyric acid, ammonium formate, trimethylamine, ammonia, or ammonium hydroxide. In some aspects, the mobile phase may be free of any additives.

In an aspect of the present invention, the column chromatography involves a mobile phase gradient. In an aspect of the present invention, the column chromatography involves purification of one or more polyunsaturated fatty acid esters. In an aspect of the present invention, the polyunsaturated fatty acid ester is a polyunsaturated fatty acid ester selected from one or more of docosahexaenoic acid, crotonic acid, myristoleic acid, palmitoleic acid, sapienic acid, oleic acid, elaidic acid, vaccenic acid, gadoleic acid, eicosenoic acid, erucic acid, nervonic acid, linoleic acid, eicosadienoic acid, docosadienoic acid, linolenic acid, pinolenic acid, eleostearic acid, mead acid, dihomo-$\gamma$-linolenic acid, eicosatrienoic acid, stearidonic acid, arachidonic acid, eicosatetraenoic acid, adrenic acid, bosseopentaenoic acid, eicosapentaenoic acid, ozubondo acid, sardine acid, tetracosapentaenoic acid, herring acid, propionic acid, butyric acid, valeric acid, caproic acid, enanthic acid, caprylic acid, pelargonic acid, capric acid, undecyl acid, lauric acid, tridecylic acid, myristic acid, pentadecylic acid, palmitic acid, margaric acid, stearic acid, nonadecylic acid, arachidic acid, henicosylic acid, behenic acid, trichosylic acid, lignoceric acid, pentacosylic acid, cerotic acid, carboceric acid, montanic acid, nonacosylic acid, melicic acid, hentriaconic acid, lacceroic acid, psyllic acid, geddic acid, celloplastic acid, hexatriacontyl acid, heptatriacontylic acid, octatriacontylic acid, nonatriacontylic acid, or tetracontylic acid. In an aspect of the present invention, the ester is a methyl, ethyl, propyl, butyl, pentyl, hexyl, heptyl, octyl, nonyl, or decyl ester. In an aspect of the present invention, the column chromatography involves purification of eicosapentaenoic acid ethyl ester.

In some aspects, the polyunsaturated fatty acid ester composition contains a target polyunsaturated fatty acid ester with a purity in the polyunsaturated fatty acid ester composition of about 50% or more, about 60% or more, about 70% or more, about 80% or more, about 85% or more, about 86% or more, about 90% or more, about 95% or more, about 96.5% or more, about 98% or more, about 99% or more, about 99.5% or more, about 99.8% or more, or about 99.9% or more, and/or less than about 97%, less than about 98%, less than about 99.0%, less than about 99.95%, or less than about 99.99%. In some aspects, the yield is 1% or more, 5% or more, 10% or more, 20% or more, 30% or more, 40% or more, 50% or more, 55% or more, 60% or more, 65% or more, 70% or more, 75% or more, or 80% or more, and/or less than 75%, less than 80%, less than 85%, less than 90%, or less than 95%. In some aspects, the recovery rate is 10% or more, 20% or more, 30% or more, 40% or more, 50% or more, 55% or more, 60% or more, 65% or more, 70% or more, 75% or more, 80% or more, 85% or more, 90% or more, 95% or more, 98% or more, 99% or more, or 99.7% or more, and/or less than 95%, less than 97%, less than 98%, less than 99%, less than 99.5%, less than 99.7%, or less than 99.9%.

The mobile phase may contain one or more of water, methanol, ethanol, acetonitrile, ethyl acetate, hexanes, dichloromethane, supercritical carbon dioxide, or any other solvent known in the art. The choice of mobile phase may require consideration of the polyunsaturated fatty acid ester to be purified and the stationary phase used. In the reversed-phase stationary phase for nonpolar polyunsaturated fatty acid esters, a polar mobile phase should be selected that is sufficient to elute the target polyunsaturated fatty acid ester but not so fast that the elution becomes too close to the leading end of the solvent.

In a certain aspect, a solvent gradient may be used for the mobile phase during elution. The main purpose of gradient elution is to elute analytes that are strongly retained in the column faster while eluting analytes that are weakly retained more slowly, so that the eluted analytes produce well-separated peaks upon detection. For example, in reversed-phase chromatography, starting with a low content of nonpolar solvent in the eluent allows weakly retained analytes to be separated. The strongly retained analytes will either remain on the top sorbent surface of the column or migrate very slowly. Increasing the amount of nonpolar components (e.g., acetonitrile) in the eluent leads to a steady increase of competition for the adsorption sites by the nonpolar solvent and enables the strongly retained components to move faster.

Thus, in the reversed-phase chromatography used for non-polar polyunsaturated fatty acid esters, the starting solvent for elution in the chromatography may contain polar solvent A, such as water, at a high percentage selected from about 100%, about 90%, about 80%, about 70%, about 60%, about 50%, about 40%, about 30%, about 20%, about 10%, or about 0%. Solvent B may be a solvent (e.g., methanol when solvent A is water) less polar than solvent A. Solvent B would constitute the remaining percentage of the mobile phase. As the column is run and the solvent is eluted through the stationary phase and the column, the gradient will result in a gradual increase in the concentration of solvent B over time. In some aspects, a single-composition solvent may be used as the mobile phase. In some aspects, examples of the single-composition solvent include one or more of water, methanol, ethanol, acetonitrile, ethyl acetate, hexanes, dichloromethane, supercritical carbon dioxide, or any other solvent known in the art, which may be used singly or may be mixed and used.

In a certain aspect, the rate of increase in solvent B over time may be constant. In a certain aspect, the gradient is absent and the mobile phase is a constant composition during elution. In a certain aspect, it is possible to use different rates of increase in the percentage of solvent B over different time ranges within the chromatographic method. In a certain aspect, the mobile phase may have a constant composition during a particular time range of the chromatographic method, while in other time ranges a gradient is included.

In a certain aspect, both the first and second column chromatography may have a constant composition, both may include a gradient, or both may have a constant composition in certain time ranges and may include a gradient in other time ranges. In a certain aspect, the first column chromatography may have a constant composition, the second column chromatography may include a gradient, or the first column chromatography may include a gradient and the second column chromatography may have a constant composition. In a certain aspect, in the case of including the third column chromatography, the third column chromatography may have a constant composition or may include a gradient.

Multiple solvents used in the mobile phase may be stored separately in a mobile phase feeding section and mixed using a pump prior to elution through the column. The mobile phase feeding section includes a mobile phase source and a solvent delivery system. Such a solvent delivery system includes a pump device such as a commercially available column chromatography pump, which delivers a solvent or mobile phase to the column. Such a pump typically provides a pulse-free flow, a flow rate in the range of 0.1 to 100 L/min, precise control of the flow rate, generation of high pressures (6000 psi or less), and corrosion- and solvent-resistant parts. The reciprocating pump includes a small chamber into which a solvent is pumped by the back-and-forth motion of a motor-driven piston. Two check valves, which open and close alternately, control the direction and flow of solvent in and out of the cylinder. The single-piston pump uses a specially designed cam that allows for very rapid refill times and produces a more continuous flow. The disadvantages of a flow pulsed by the reciprocating pump are often overcome by using a pulse damper. The use of dual-piston pump, which operates with pistons that move in different phases relative to each other, gives a reasonable solution for pulse-free fluid delivery. The linear velocity in the column represents a rate at which the fluid passes through the column cross-section. The linear velocity (Linear velocity (m/hour) = Flow rate ($m^3$/hour)/Column cross sectional area ($m^2$)) may be about 0.2 to 20.0 m/hour, about 1.0 to 15.0 m/hour, about 1.0 to 10.0 m/hour, about 1.5 to 10.0 m/hour, or about 2.0 to 9.0 m/hour. In an aspect, the linear velocity is about 4.0 to 9.0 m/hour.

In an aspect of the present invention, the linear velocities in the first column and in the second column may be the same or different. In the case of different linear velocities, the linear velocity in the first column may be faster or slower than the linear velocity in the second column. The ratio between the linear velocity in the first column and the linear velocity in the second column when the linear velocity in the first column is set to 1 is from 0.5 to 2.0, from 0.5 to 1.5, or from 1 to 1.5 for the second column. In an aspect of the present invention, the first and second columns have the same linear velocity. In an aspect of the present invention, when the third column chromatography is performed, the third column may have the same linear velocity as or a different linear velocity from that of the respective first or second column.

In an aspect of the present invention, the column chromatography is performed at room temperature or at a temperature higher than room temperature. Preferably, the method is implemented at a temperature higher than room temperature. The first and second column chromatography may be performed at the same or different temperatures, but preferably the same temperature.

In an aspect of the present invention, the temperature higher than room temperature is 20°C or higher, 25°C or higher, 30°C or higher, or 35°C or higher, or 60°C or lower, 55°C or lower, 50°C or lower, or 45°C or lower, or from 20 to 60°C, from 25 to 55°C, from 30 to 50°C, or from 35 to 45°C.

In an aspect of the present invention, two or more columns may be used for the column chromatography. The column chromatography may be performed using any known fixed-bed chromatography system. Such column chromatography is called fixed-bed chromatography. In an aspect of the present invention, the first and second column chromatography are fixed-bed chromatography.

In some aspects, a detector is used to monitor the mobile phase eluting from the column for the presence of component(s). Detection methods known in the art (e.g., mass spectrometry (MS), UV/Vis absorbance, fluorescence, refractive index, conductivity) may be used.

In other aspects, any of a variety of standard column chromatography detectors can be used for detection of eluate immediately after elution from the column.

In other aspects, each fraction may be monitored and analyzed for the presence of component(s). In some aspects, the analysis is fatty acid analysis using gas chromatography.

In some aspects, the eluate from the column is detected as a peak in the chromatogram. The retention time at the peak is used to identify a compound, and the height (or area) of the peak is proportional to the amount of eluate in the fatty oil composition. The "retention time" is a time required for the eluate to pass through the column and is measured from the time point of injecting (or loading) the fatty oil composition to the time point of elution. Ideally, each eluate of interest would have a characteristic retention time. However, the retention of the eluate varies when the eluent, stationary phase, temperature, and column chromatography setting conditions are changed. Thus, the retention time of the eluate is compared with the retention times of one or more standard compounds under the same conditions. Suitable detectors exhibit good sensitivity, good stability, reproducibility, a linear response when used for quantitative purposes over several orders of digits, a short response time, and easy-to-operate property. Examples of such detectors include, but are not limited to, UV/Vis absorbance detectors, photodiode array detectors, fluorescence detectors, refractive index detectors, and conductivity detectors.

A UV/Vis absorbance detector including a scanning spectrophotometer with grid optics may be used. The use of a deuterium source (UV range, 190-360 nm) independently or in combination with a tungsten source (visible range, 360-800 nm) provides a simple means of detecting an absorbing species when discharged from the column.

A photodiode array (PDA)-based instrument is a UV/visible absorbance detector that allows for very rapid collection of data over a selected spectral range. Absorbance spectral data for each chromatographic peak can be collected and stored. The stored data can be compared with spectra of pure standards from the library. The PDA detector is useful in identifying components (overlapping peaks) that are difficult to separate because the characteristic spectra for the respective inseparable components are likely to be different.

Fluorescence detectors are useful for the detection of analytes that exhibit light-emitting chemical properties such as fluorescence or phosphorescence. They are at least one order of magnitude more sensitive than UV absorbance detectors. Fluorescence is observed by detecting grid-separated emission radiation typically at an angle of 90 degrees to the excitation beam. The number of fluorescent species can be enhanced by a post-column derivatization (PCD) reaction of eluted compound(s) (or pre-column derivatization reaction of the sample itself) while using a special reagent.

The refractive index (RI) detector responds to almost all solutes. The difference in the refractive index of the reference mobile phase relative to the column effluent results in the detection of the separated component(s) as a peak(s) on the resulting chromatogram. Due to its extremely high sensitivity to the mobile phase, this detector cannot be used without sufficient pulse attenuation in the LC pump, and it is not even suitable for gradient application due to the changing mobile phase composition. The detection limit is usually lower than that observed with an absorbance detector.

The conductivity detector provides highly sensitive detection of any charged species. This detector can be used with an LC system for simple and reliable detection of anions, cations, metals, organic acids, and surfactants down to ppb levels. The addition of a chemical inhibitor between the column and the conductivity detector serves to reduce the conductivity of the eluent, allowing for the use of gradient elution and the determination at ppb levels with minimal baseline drift. For typical determination of low-level anions, the eluent is converted to its weakly ionized, low-conductivity acid (e.g., $Na_2CO_3$ to carbonic acid), thereby reducing background noise. At the same time, the analyte anions are converted to their corresponding high-conductivity acids (e.g., NaCl to HCl), thereby increasing the relative analyte signal.

In an aspect of the present invention, the purity of polyunsaturated fatty acid ester in the polyunsaturated fatty acid ester composition obtained by the second column chromatography is 93 wt% or more, 95 wt% or more, 96 wt% or more, 96.5 wt% or more, 97 wt% or more, 97.5 wt% or more, 98 wt% or more, 98.5 wt% or more, 99 wt% or more, or 99.5 wt% or more based on the total polyunsaturated fatty acid ester composition.

The retention time of the target substance may be about 0.5 to about 2 minutes, about 2 to about 4 minutes, about 4 to about 6 minutes, about 6 to about 8 minutes, about 8 to about 10 minutes, about 10 to about 12 minutes, about 12 to about 14 minutes, about 14 to about 16 minutes, about 16 to about 18 minutes, about 18 to about 20 minutes, about 20 to about 22 minutes, about 22 to about 24 minutes, about 24 to about 26 minutes, about 26 minutes to about 28 minutes, about 28 minutes to about 30 minutes, about 30 minutes to about 32 minutes, about 32 minutes to about 34 minutes, about 34 minutes to about 36 minutes, about 36 minutes to about 38 minutes, or about 38 minutes to about 40 minutes, or longer than about 0.5 minutes, longer than about 1 minute, longer than about 1.5 minutes, longer than about 2 minutes, or longer than about 5 minutes, and/or less than 50 minutes, less than 40 minutes, less than 30 minutes, or less than 20 minutes.

In an aspect of the present invention, the retention time at the peak top of the target substance in the first column chromatography is shorter than the retention time at the peak top of the target substance in the second column chromatography. The retention time at the peak top of the target substance in the first column chromatography may be about 0.5 to about 2 minutes, about 2 to about 4 minutes, about 4 to about 6 minutes, about 6 to about 8 minutes, or about 8 to about 10 minutes, or less than 10 minutes, less than 9 minutes, less than 8 minutes, less than 7 minutes, less than 6 minutes, less than 5 minutes, less than 4 minutes, less than 3 minutes, less than 2 minutes, or less than 1 minute. The retention time at the peak top of the target substance in the second column chromatography may be about 6 minutes to about 8 minutes, about 8 minutes to about 10 minutes, about 10 minutes to about 12 minutes, about 12 minutes to about 14 minutes, about 14 minutes to about 16 minutes, about 16 minutes to about 18 minutes, about 18 minutes to about 20 minutes, about 20 minutes to about 22 minutes, about 22 minutes to about 24 minutes, about 24 minutes to about 26 minutes, about 26 minutes to about 28 minutes, about 28 minutes to about 30 minutes, about 30 minutes to about 32 minutes, about 32 minutes to about 34 minutes, about 34 minutes to about 36 minutes, about 36 minutes to about 38 minutes, or about 38 minutes to about 40 minutes, or less than 40 minutes, less than 38 minutes, less than 36 minutes, less than 34 minutes, less than 32 minutes, less than 30 minutes, less than 28 minutes, less than 26 minutes, less than 24 minutes, less than 22 minutes, less than 20 minutes, less than 18 minutes, less than 16 minutes, less than 14 minutes, less than 12 minutes, less than 10 minutes, or less than 8 minutes.

In an aspect of the present invention, an internal standard may be used during analysis by gas chromatography (GC). The internal standard may be added to the sample as a reference marker to determine the retention time of the analyte relative to the internal standard or to assist in quantification of the analyte. The internal standard may be selected, if appropriate, by a person skilled in the art to be a compound very similar but not identical to the target analyte, for example, a deuterated derivative of the target analyte. When used for quantification purpose, the internal standard can then be used for calibration by plotting the ratio of the signal of the analyte to the signal of the internal standard as a function of the analyte concentration with respect to the standard concentration. Here, the standard is a sample at a known concentration as prepared by a person skilled in the art to be used as a reference for a sample containing an unknown analyte species to be quantified.

In a certain aspect, the polyunsaturated fatty acid ester is or contains docosahexaenoic acid, crotonic acid, myristoleic acid, palmitoleic acid, sapienic acid, oleic acid, elaidic acid, vaccenic acid, gadoleic acid, eicosenoic acid, erucic acid, nervonic acid, linoleic acid, eicosadienoic acid, docosadienoic acid, linolenic acid, pinolenic acid, eleostearic acid, mead acid, dihomo-γ-linolenic acid, eicosatrienoic acid, stearidonic acid, arachidonic acid, eicosatetraenoic acid, adrenic acid, bosseopentaenoic acid, eicosapentaenoic acid, ozubondo acid, sardine acid, tetracosapentaenoic acid, herring acid, propionic acid, butyric acid, valeric acid, caproic acid, enanthic acid, caprylic acid, pelargonic acid, capric acid, undecyl acid, lauric acid, tridecylic acid, myristic acid, pentadecylic acid, palmitic acid, margaric acid, stearic acid, nonadecylic acid, arachidic acid, henicosylic acid, behenic acid, trichosylic acid, lignoceric acid, pentacosylic acid, cerotic acid, carboceric acid, montanic acid, nonacosylic acid, melicic acid, hentriaconic acid, lacceroic acid, psyllic acid, geddic acid, celloplastic acid, hexatriacontyl acid, heptatriacontylic acid, octatriacontylic acid, nonatriacontylic acid, or tetracontylic acid, or an ester thereof.

In a certain aspect, the purity of the polyunsaturated fatty acid ester composition may be greater than about 70%, greater than about 80%, greater than about 85%, greater than about 86%, greater than about 90%, greater than about 95%, greater than about 96.5%, greater than about 98%, greater than about 99%, greater than about 99.5%, greater than about 99.8%, or greater than about 99.9%. In a certain aspect, the purity of the polyunsaturated fatty acid ester composition may be less than about 100%, less than about 99.9999%, less than about 99.5%, less than 99%, less than 98.5%, or less than about 98%. In a certain aspect, the purity of the polyunsaturated fatty acid ester composition may be 70-98%, 80-98%, 85-98.5%, 86-98.5%, 90-98.5%, 95-99%, 96.5-99%, 98-99.5%, 99-99.5%, 99.5-99.9999%, 99.8-99.9999 %, or 99.8-100%. In a certain aspect, the yield of the polyunsaturated fatty acid ester composition may be greater than 50%, greater than 55%, greater than 60%, greater than 65%, greater than 70%, greater than 75%, or greater than 80%. In a certain aspect, the yield of the polyunsaturated fatty acid ester composition may be less than 100%, less than 95%, less than 90%, or less than 80%. In a certain aspect, the yield of the polyunsaturated fatty acid ester composition may be 50-80%, 55-90%, 60-90%, 65-95%, 70-95%, 75-100%, or 80-100%. In a certain aspect, the recovery rate of the polyunsaturated fatty acid ester composition may be greater than 55%, greater than 60%, greater than 65%, greater than 70%, greater than 75%, greater than 80%, greater than 85%, greater than 90%, greater than 95%, greater than 98%, greater than 99%, or greater than 99.7%. In a certain aspect, the recovery rate of the polyunsaturated fatty acid ester composition may be less than 100% or less than 99.9999%. In a certain aspect, the purity of the polyunsaturated fatty acid ester composition may be 55-99.9999%, 60-99.9999%, 65-99.9999%, 70-99.9999%, 75-99.9999%, 80-99.9999%, 85-99.9999%, 90-99.9999%, 95-99. 9999%, 98-99.9999%, 99-100%, or 99.7-100%.

In an aspect of the present invention, the purity of the polyunsaturated fatty acid ester in the polyunsaturated fatty acid ester composition obtained by the second column chromatography is an EPA ester (e.g., an EPA ethyl ester), and the purity is 93 wt% or more, 95 wt% or more, 96 wt% or more, 96.5 wt% or more, 97 wt% or more, 97.5 wt% or more, 98 wt% or more, 98.5 wt% or more, 99 wt% or more, or 99.5 wt% or more based on the total polyunsaturated fatty acid ester composition.

In an aspect of the present invention, the purity of the polyunsaturated fatty acid ester in the polyunsaturated fatty acid ester composition obtained by the second column chromatography is a DGLA ester (e.g., a DGLA ethyl ester) is 93 wt% or more, 95 wt% or more, 96 wt% or more, 96.5 wt% or more, 97 wt% or more, 97.5 wt% or more, 98 wt% or more, 98.5 wt% or more, 99 wt% or more, or 99.5 wt% or more based on the total polyunsaturated fatty acid ester composition.

In an aspect of the present invention, the polyunsaturated fatty acid ester obtained by the second column chromatography is an ARA ester (e.g., an ARA ethyl ester), and the purity of the polyunsaturated fatty acid ester is high and is 93 wt% or more, 95 wt% or more, 96 wt% or more, 96.5 wt% or more, 97 wt% or more, 97.5 wt% or more, 98 wt% or more, 98.5 wt% or more, 99 wt% or more, or 99.5 wt% or more based on the total polyunsaturated fatty acid ester composition.

In an aspect of the present invention, the polyunsaturated fatty acid composition obtained through the second column chromatography contains at least one fatty acid ester selected from the group consisting of C18:0, C20:0, C20:1, and C22:0 esters in an amount of 0.6 wt% or less, 0.3 wt% or less, 0.2 wt% or less, 0.1 wt% or less, 0.05 wt% or less, 0.03 wt% or less, or 0.01 wt% or less based on the total polyunsaturated fatty acid ester composition.

Hereinbelow, Examples of the present invention will be described. However, the present invention is not at all limited to them.

EXAMPLES

[Example 1]

In this study, whether the loading interval was shortened was checked in a column model by comparing a single series column with a double series column. A sample containing EPA-EE, C20:0 ethyl ester, and C20:1 ethyl ester was prepared from crude refined sardine oil (preparation method was the same as in Example 2 below). The single series column used consisted of columns each with an inner diameter of 20 mm and a length of 300 mm, 500 mm, and 500 mm as connected in series. The double series column used consisted of the first column with an inner diameter of 20 mm and a length of 300 mm and the second column having two columns with an inner diameter of 20 mm and a length of 500 mm as connected in series. The column temperature was 40°C. The flow rate of the single series column was 37 mL/min. The flow rate of the first column in the double series column was 32 mL/min and the flow rate of the second column was 37 mL/min. Daisopak SP-120-50-ODS-B (OSAKA SODA) was used as the stationary phase. The mobile phase was HPLC grade methanol (KANTO CHEMICAL CO., INC.). Detection was by UV at 230 nm.

In the single series column, EPA-EE started eluting 13 minutes after the start of column chromatography. The elution of C20:0 ethyl ester ended 24 minutes after the start of elution of EPA-EE.

In the double series column, the elution of EPA-EE from the first column ended 3.5 minutes after the start of column chromatography. In the second column, the elution of EPA-EE started 10.5 minutes after the start of column chromatography. The elution of C20:1 ethyl ester ended 12 minutes after the start of elution of EPA-EE.

In the single series column, the fatty acid farthest from EPA-EE is C20:0 ethyl ester. In the double series column, an eluent of up to 3.5 minutes, which is the end time of EPA-EE elution in the first column, was collected and loaded onto the second column. In this case, the component that is the furthest away from EPA-EE in the second column is fatty acid C20:1 ethyl ester. This is because the C20:0 ethyl ester has been removed by the first column. The elution of C20:0 ethyl ester ended 24 min after the start of EPA-EE elution in the single series column. Thus, by setting the loading interval to 24 minutes, the C20:0 ethyl ester that was the last to be eluted in the previous loading would not be mixed into the fraction of EPA-EE detected in the next loading.

By contrast, in the double series column, the elution of C20:1 ethyl ester ended 12 min after the start of elution of EPA-EE in the second column. Thus, if the loading interval is every 12 minutes, the C20:1 ethyl ester that was the last to be eluted in the previous loading would not be mixed with the fraction of EPA-EE eluted after the next loading.

In other words, the loading interval in the single column was reduced by half, from 24 minutes to 12 minutes, by introducing the double series column. This was found to be an effective way to reduce the solvent used.

[Example 2]

The conditions of column chromatography were as described below. The HPLC equipment used was as follows. The pump was NP-KX500 (Nihon Seimitsu Kagaku Co., Ltd.), the detector was S-3702 (SOMA OPTICS, LTD.), and the column oven was CO705 (GL Sciences Inc.). The ODS filler used was Daisopak SP-120-50-ODS-B (OSAKA SODA). The first column had an inner diameter of 20 mm and a length of 300 mm, and the second column had an inner diameter of 20 mm and a length of 1000 mm. The flow rate of the first column was 32 mL/min and the flow rate of the second column was 37 mL/min. The solvent was HPLC grade methanol (KANTO CHEMICAL CO., INC.). Detection was by UV at 230 nm. Here, 2.4 g of EPA-EE (80%) was loaded.

EPA-EE (80%) was prepared as follows. Short process distillation (SPD) was performed on crude refined sardine oil. An ethanolysis reaction with ethyl alcohol was performed using SPD-treated oil in the presence of an alkali catalyst to form EPA-EE, a fish oil ethyl ester. Here, the fish oil ethyl ester was subjected to fine distillation to prepare 80% EPA-EE.

First, a fractionation test was performed on the first column. The first column was loaded with 2.4 g of EPA-EE (80%), and a fraction from immediately after injection until when the UV value increased was fractionated as Fr. 0, followed by fractionation of Fr. 1 to 20 for 10 seconds each, and then fractionation of Fr. 29 for 30 minutes. Methanol was removed from the fractionated fraction by using a vacuum evaporator; a hexane solution containing 1 mg/mL C23:0 methyl ester was added as an internal standard; and the sample was then analyzed by gas chromatography (GC). The amount of elution of compound in each fraction was calculated from the area ratio between the GC peak area and the internal standard area. As a result, the elution of EPA-EE (93% content) started at 3 minutes and 30 seconds, and ended at 4 minutes and 20 seconds. The analogue composition at that time was then determined. Thus, the eluent in the range between 3 minutes 30 seconds and 4 minutes 20 seconds from the start of column chromatography was determined to be fed from the first column to the second column.

Next, after 2.4 g was loaded onto the first column, the eluent that was in the range (between 3 minutes 30 seconds and 4 minutes 20 seconds) and contained 93% or more EPA-EE was fed to the second column. A fraction of the eluent until when the UV value increased in the second column was set as Fr. 0, Fr. 1-38 were fractionated for 10 seconds each, followed by fractionation of Fr. 39 for 35 minutes. Similar to the fractionation test of the first column, GC analysis was performed to determine the analogue composition of when EPA-EE was 97% or higher.

The conditions used for the GC analysis were as follows. The instrument was a 7890A network GC system (Agilent) with a DB-WAX 30 m × 0.25 mm × 0.25 μm column. The column temperature was 210°C. The injection temperature was 250°C, the split rate was 1:50, and the injection volume was 1 μL. A FID detector at 250°C was used. A helium carrier gas with a linear velocity of 31 cm/min was used.

[Table 1]

| EPA Data (ODS-B) | | | | | | | |
|---|---|---|---|---|---|---|---|
| | %Fatty acid before first column | First elution time [min] (peak top value) | %Fatty acid after first column | First elution time relative value (peak top value) | Second elution time (peak top value) | %Fatty acid collected through second column | Second elution time relative value (peak top value) |
| C18:4 | 0.04% | 3.7 | 0.03% | 0.94 | 14.83 | 0.14% | 0.97 |
| EPA | 81.75% | 3.9 | 93.72% | 1.00 | 15.33 | 97.68% | 1.00 |
| C19:4 | 0.04% | 3.9 | 0.05% | 1.00 | 15.83 | 0.03% | 1.03 |
| C21:5 | 0.09% | 4.0 | 0.07% | 1.03 | 16.67 | 0.06% | 1.09 |
| C18:0 | 0.58% | 7.3 | 0.00% | 1.89 | - | 0.00% | - |
| C20:1 | 4.05% | 7.3 | 0.00% | 1.89 | - | 0.00% | - |
| C20:0 | 0.25% | 9.0 | 0.00% | 2.32 | - | 0.00% | - |

[Example 3]

The conditions of column chromatography for DGLA concentration were as described below.

The column was an axial compression column LC-600 (Kurita Water Industries, Ltd.) with an inner diameter of 600 mm was packed at a packing pressure of 3.5 MPa with an ODS filler (SP-120-50-ODS-B, OSAKA SODA) having a particle size of 50 μm in an amount of 75 kg for the first column and an amount of 115 kg for the second column. The first column and the second column had a column length of 50 cm and 80 cm, respectively. Methanol was used as the mobile phase and the flow rate was 28 L/min for both the first and second columns.

Fatty oil composition 1 (DGLA content: 87.7%) was prepared as follows. Specifically, microbial oil 1 derived from a microorganism Mortierella sp. containing 37.2 wt% DGLA in a fatty acid composition was ethyl-esterified with an alkali catalyst according to a routine procedure, and the C20 fraction was concentrated by fine distillation to obtain fatty oil composition 1.

First, fractionation was performed on the first column. The first column was loaded with 8.6 kg of the fatty oil composition 1 (DGLA content: 87.7%) (4.5% of the weight of adsorbent in the first and second columns). A fraction (Fr. 1) was fractionated from immediately after the end of injection. Fr. 1 was fractionated for 180 seconds, each of Fr. 2 to Fr. 19 was fractionated for 30 seconds, and each of Fr. 20 to Fr. 33 was fractionated for 120 seconds. Here, 1 mL of each fractionated fraction was aliquoted into a Falcon tube. A whole pipette was used to add 1 mL of hexane solution containing 1 mg/mL methyl tricosanoate. In addition, 5 mL of 5% NaCl solution was added and stirred vigorously for separation into two layers. The upper layer was then collected in a GC vial and provided as a GC sample for GC-FID analysis. The GC conditions were as described below. The amount of elution of compound in each fraction was calculated from the area ratio between the GC peak area and the internal standard area.

<<GC Analytical Conditions>>

Instrument: capillary gas chromatograph GC-2025, Shimadzu Corporation
Column: DB-WAX (30 m × 0.25 mm ID; film thickness: 0.25 μm)
Carrier gas: hydrogen, 1 mL/min
Split ratio: 1:30
Column temperature: 230°C (30 min)
Injection inlet temperature: 250°C
Detector type, temperature: FID, 250°C
Injection volume: 1 μL

As a result, the elution of the target substance DGLA in the first column started at 4.5 minutes and ended at 30 minutes. The start time and end time of elution of the target fraction (DGLA content: 92.7%) to be fed to the second column were intentionally set to 4.5 minutes and 9 minutes, respectively, so that C22:0, which takes a long time to elute, was suppressed to 0.0%.

Next, after the loading of 8.6 kg of fatty oil composition 1 onto the first column was completed, the time during which the eluent would be fed from the first column to the second column was set to the range (from 4.5 to 9 minutes) specified in the fractionation through the first column described above. Fractionation of an eluent from the second column was started immediately after the fatty oil composition 1 was fed from the first column to the second column, and Fr. 1 was fractionated for 360 seconds, each of Fr. 2 to Fr. 24 was fractionated for 30 seconds, and each of Fr. 25 to Fr. 34 was fractionated for 120 seconds. GC analysis was performed as in the fractionation test for the first column. The recovered fractions were identified so that the DGLA content in the fatty acid composition of the recovered fractions was 96.5% or higher. The fatty acid composition of the recovered fractions at that time was then determined.

[Table 2]

| Fatty acid | Fatty oil composition 1 | Elution time [min] (Peak top value First) | Collected through first column | Second column injection | Elution time [min] (Peak top value Second) | After second column |
|---|---|---|---|---|---|---|
| DGLA | 87.74% | 6 | 92.74% | As described in the left | 18 | 96.54% |
| C20:4n-6 | 0.66% | 5.5 | 0.64% | As described in the left | 17.5 | 0.54% |
| C22:0 | 1.74% | 18 | 0.02% | As described in the left | - | 0 |

[Example 4]

The conditions of column chromatography for ARA concentration were as described below.

The HPLC column and mobile phase conditions were the same settings as those used in Example 2.

Fatty oil composition 2 (ARA content: 79.4%) was prepared as follows.

Specifically, microbial oil 2 derived from a microorganism Mortierella sp. containing 43.6 wt% ARA in a fatty acid composition was ethyl-esterified with an alkali catalyst according to a routine procedure, and the C20 fraction was concentrated by fine distillation to obtain fatty oil composition 2.

First, fractionation was performed on the first column. The first column was loaded with 1.0 kg of the fatty oil composition 2 (ARA content: 79.4%) (0.55% of the total weight of adsorbent in the first and second columns). A fraction (Fr. 1) was fractionated from immediately after the end of injection. Fr. 1 was fractionated for 361 seconds, each of Fr. 2 to Fr. 13 was fractionated for 30 seconds, each of Fr. 14 to Fr. 16 was fractionated for 300 seconds, and each of Fr. 17 to Fr. 29 was fractionated for 60 seconds. Each fractionated fraction was subjected to GC analysis using the same method as in Example 2, and the amount of elution of compound in each fraction was determined. As a result, the elution of the target component ARA, after the first column, started at 6 minutes and ended at 40 minutes. The start time and end time of elution of the target fraction (ARA content: 78.9%) to be fed to the second column were intentionally set to 6.5 minutes and 12 minutes, respectively, so that C22:0, which takes a long time to elute, was suppressed to 0.0%.

Next, after the loading of 1.0 kg of fatty oil composition 2 onto the first column was completed, the time during which the eluent would be fed from the first column to the second column was set to the range (from 6.5 to 12 minutes) specified in the fractionation through the first column described above. Fractionation of an eluent from the second column was started immediately after the fatty oil composition 2 was loaded onto the first column, and Fr. 1 was fractionated for 1051 seconds, each of Fr. 2 to Fr. 31 was fractionated for 10 seconds, and each of Fr. 32 to Fr. 33 was fractionated for 600 seconds. A GC sample prepared by the same pretreatment as in the fractionation test for the first column was analyzed under the following GC conditions. The recovered fractions were identified so that the ARA content in the fatty acid composition of the recovered fractions was 96.5% or higher. The fatty acid composition of the recovered fractions at that time was then determined.

<<GC Analytical Conditions>>

Instrument: capillary gas chromatograph GC-2025, Shimadzu Corporation
Column: DB-WAX (30 m $\times$ 0.25 mm ID; film thickness: 0.25 $\mu$m)
Carrier gas: hydrogen, controlled at a constant linear velocity of 31.8 cm/sec

Split ratio: 1:30
Column temperature: 210°C (30 min)
Injection inlet temperature: 250°C
Detector type, temperature: FID, 250°C
Injection volume: 1 μL

[Table 3]

| Fatty acid | Fatty oil composition 2 | Elution time [min] (Peak top value First) | % after first column | % at second column injection | Elution time [min] (Peak top value Second) | % After second column |
|---|---|---|---|---|---|---|
| ARA | 79.42% | 8.0 | 78.86 | As described in the left | 19.9 | 97.16 |
| C18:2n-6 | 2.24% | 8.0 | 2.97 | As described in the left | 21.2 | 1.91 |
| C18:3n-6 | 0.32% | 7.5 | 0.44 | As described in the left | 18.9 | 0.19 |
| C18:3n-3 | 0.14% | 7.5 | 0.21 | As described in the left | 19.0 | 0.09 |
| C22:0 | 0.69% | 22.0 | 0.00 | As described in the left | - | 0.00 |

## Claims

1. A method for producing a polyunsaturated fatty acid ester composition from a fatty oil composition, the method comprising:

    (a) subjecting the fatty oil composition to a first column chromatography using a first column to remove all or part of at least one component, wherein a retention time at a peak top of the at least one component corresponds to a relative value of 1.8 to 3.5, wherein the relative value is based on a retention time at a peak top of a target substance in the first column chromatography which is set to 1.0, wherein the target substance is a polyunsaturated fatty acid ester;
    (b) subjecting an eluent containing the target substance obtained by the first column chromatography to a second column chromatography using a second column to remove all or part of at least one component, wherein a retention time at a peak top of the at least one component corresponds to a relative value of 0.90 to 1.1, wherein the relative value is based on a retention time at a peak top of the target substance in the second column chromatography which is set to 1.0; and
    (c) concentrating an eluent obtained by the second column chromatography to obtain a composition containing the target substance such that the obtained composition contains the target substance in an amount of 95 wt% or higher.

2. The method according to claim 1, wherein a loading interval is shorter than in a case of column chromatography using a single column with a length that is a sum of lengths of the first and second columns, wherein the loading interval is a period from starting one run of column chromatography to reaching a state wherein starting a subsequent run of column chromatography becomes possible.

3. The method according to claim 1 or 2, wherein the at least one component having the retention time at the peak top corresponding to a relative value of 1.8 to 3.5 is at least one component other than the polyunsaturated fatty acid ester in the fatty oil composition, wherein the relative value is based on a retention time at a peak top of the target substance in the first column chromatography which is set to 1.0.

4. The method according to any one of claims 1 to 3, wherein the all or part of the at least one component that is removed in step (b), is at least one component having a retention time at a peak top corresponding to a relative value of 0.93 to 1.09, wherein the relative value is based on a retention time at a peak top of the target substance in the second column chromatography which is set to 1.0.

5. The method according to any one of claims 1 to 4, wherein the first column can be switched between a connected state with the second column so that the eluent from the first column is injected into the second column and a non-connected state with the second column.

6. The method according to any one of claims 1 to 5, wherein the first column chromatography and the second column chromatography are each reversed-phase column chromatography.

7. The method according to any one of claims 1 to 6, wherein the first column chromatography and the second column chromatography are each fixed-bed column chromatography.

8. The method according to any one of claims 1 to 7, wherein the first column chromatography and the second column chromatography use an identical mobile phase.

9. The method according to any one of claims 1 to 8, wherein the second column has a larger amount of packed stationary phase than the first column, or has the same inner diameter as the first column and is longer than the first column.

10. The method according to any one of claims 1 to 9, wherein the target substance is an ester of any of eicosapentaenoic acid (EPA), dihomo-$\gamma$-linolenic acid (DGLA), or arachidonic acid (ARA).

11. The method according to any one of claims 1 to 10, wherein the at least one component, all or part of which is removed by the first column chromatography, is an ester of any of C22:0, C20:0, C18:0, or C20:1.

12. The method according to claim 10 or 11, wherein the ester is an ethyl ester.

13. The method according to any one of claims 1 to 12, wherein the polyunsaturated fatty acid ester composition contains the target substance in an amount of 96 wt% or more.

14. The method according to any one of claims 1 to 13, wherein steps (a), (b) and (c) are repeated twice or more.

15. The method according to any one of claims 1 to 14, wherein the target substance is an eicosapentaenoic acid ester and the at least one component, all or part of which is removed by the second column chromatography, is an ester of stearidonic acid, C19:4, or C21:5.

16. The method according to any one of claims 1 to 14, wherein the target substance is a dihomo-$\gamma$-linolenic acid ester and the at least one component, all or part of which is removed by the second column chromatography, is an ester of C20:4n-6.

17. The method according to any one of claims 1 to 14, wherein the target substance is an arachidonic acid ester and the at least one component, all or part of which is removed by the second column chromatography, is an ester of C18:3n-6.

## INTERNATIONAL SEARCH REPORT

| International application No. |
| --- |
| **PCT/JP2024/022429** |

**A. CLASSIFICATION OF SUBJECT MATTER**

*C11B 7/00*(2006.01)i; *A23D 9/02*(2006.01)i; *A23L 33/12*(2016.01)i; *A61K 8/37*(2006.01)i; *A61K 31/232*(2006.01)i; *B01D 15/32*(2006.01)i; *C11B 3/10*(2006.01)i; *G01N 30/46*(2006.01)i; *G01N 30/84*(2006.01)i; *G01N 30/86*(2006.01)i; *G01N 30/88*(2006.01)i

FI: C11B7/00; A23D9/02; A23L33/12; A61K8/37; A61K31/232; B01D15/32; C11B3/10; G01N30/46 A; G01N30/84 Z; G01N30/86 M; G01N30/88 C; G01N30/88 E

According to International Patent Classification (IPC) or to both national classification and IPC

**B. FIELDS SEARCHED**

Minimum documentation searched (classification system followed by classification symbols)

C11B7/00; A23D9/02; A23L33/12; A61K8/37; A61K31/232; B01D15/32; C11B3/10; C11C1/00; G01N30/46; G01N30/84; G01N30/86; G01N30/88

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2017-504683 A (NOVASEP PROCESS) 09 February 2017 (2017-02-09) claim 1, paragraphs [0011], [0048], [0053], [0056], [0067], [0088], [0101], [0152], [0154] | 1-17 |
| A | US 5130449 A (NESTEC S.A.) 14 July 1992 (1992-07-14) entire text | 1-17 |
| A | JP 2014-525951 A (BASF PHARMA (CALLANISH) LIMITED) 02 October 2014 (2014-10-02) entire text, all drawings | 1-17 |
| A | WO 2017/191821 A1 (NISSHIN PHARMA INC.) 09 November 2017 (2017-11-09) entire text, all drawings | 1-17 |

☑ Further documents are listed in the continuation of Box C.    ☑ See patent family annex.

| | |
| --- | --- |
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **27 August 2024** | **10 September 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

| International application No. |
| --- |
| **PCT/JP2024/022429** |

**C.      DOCUMENTS CONSIDERED TO BE RELEVANT**

| Category* | Citation of document, with indication, where appropriate, of the relevant passages | Relevant to claim No. |
| --- | --- | --- |
| A | JP 2008-539395 A (EIDGENOESSISCHE TECHNISCHE HOCHSCHULE ZUERICH) 13 November 2008 (2008-11-13)<br>    entire text, all drawings | 1-17 |
| A | Microfilm of the specification and drawings annexed to the request of Japanese Utility Model Application No. 184117/1985 (Laid-open No. 092470/1987) (DAICEL CHEMICAL INDUSTRIES, LTD.) 12 June 1987 (1987-06-12), entire text, all drawings | 1-17 |

Form PCT/ISA/210 (second sheet) (July 2022)

## INTERNATIONAL SEARCH REPORT
### Information on patent family members

International application No.

**PCT/JP2024/022429**

| Patent document cited in search report | | | Publication date (day/month/year) | Patent family member(s) | | | Publication date (day/month/year) |
|---|---|---|---|---|---|---|---|
| JP | 2017-504683 | A | 09 February 2017 | US | 2016/0304433 | A1 | |
| | | | | claim 1, paragraphs [0013], [0077], [0082], [0085], [0096], [0123], [0136], [0190], [0192] | | | |
| | | | | WO | 2015/086591 | A1 | |
| | | | | EP | 3079787 | A1 | |
| | | | | KR | 10-2016-0096627 | A | |
| | | | | CN | 105848747 | A | |
| US | 5130449 | A | 14 July 1992 | EP | 399417 | A3 | |
| JP | 2014-525951 | A | 02 October 2014 | US | 2014/0200360 | A1 | |
| | | | | entire text, all drawings | | | |
| | | | | WO | 2013/005052 | A1 | |
| | | | | EP | 2613861 | A1 | |
| | | | | CN | 103764241 | A | |
| | | | | KR | 10-2014-0034924 | A | |
| WO | 2017/191821 | A1 | 09 November 2017 | US | 2019/0144780 | A1 | |
| | | | | entire text, all drawings | | | |
| | | | | EP | 3453749 | A1 | |
| | | | | CN | 109072126 | A | |
| JP | 2008-539395 | A | 13 November 2008 | US | 2009/0050567 | A1 | |
| | | | | entire text, all drawings | | | |
| | | | | WO | 2006/116886 | A1 | |
| | | | | EP | 1716900 | A1 | |
| | | | | CN | 101166564 | A | |
| JP | 62-092470 | U1 | 12 June 1987 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014054435 A
- JP 2014511406 W
- JP H08512336 W
- JP H08218091 A
- JP 2016508156 W
- JP 2019207234 A
- JP 2013516398 W